(19) Europäisches Patentamt
European Patent Office
Office européen des brevets

(11) EP 0 736 011 B1

(12) # EUROPÄISCHE PATENTSCHRIFT

(45) Veröffentlichungstag und Bekanntmachung des Hinweises auf die Patenterteilung:
**26.07.2000 Patentblatt 2000/30**

(21) Anmeldenummer: **95903342.4**

(22) Anmeldetag: **14.12.1994**

(51) Int. Cl.$^7$: **C07D 217/14**, C07D 217/16, C07D 217/18, C07D 409/06, C07D 409/12, A61K 31/47

(86) Internationale Anmeldenummer:
**PCT/EP94/04150**

(87) Internationale Veröffentlichungsnummer:
**WO 95/17389 (29.06.1995 Gazette 1995/27)**

(54) **ANELLIERTE DIHYDROPYRIDINE UND DEREN VERWENDUNG FÜR DIE HERSTELLUNG VON PHARMAZEUTISCHEN ZUBEREITUNGEN**

ANELLATED DIHYDROPYRIDINES AND THEIR USE IN THE PRODUCTION OF PHARMACEUTICAL PREPARATIONS

DIHYDROPYRIDINES ANNELEES ET LEUR UTILISATION POUR LA PRODUCTION DE PREPARATIONS PHARMACEUTIQUES

(84) Benannte Vertragsstaaten:
**AT BE CH DE DK ES FR GB GR IE IT LI LU MC NL PT SE**

(30) Priorität: **21.12.1993 DE 4343683**

(43) Veröffentlichungstag der Anmeldung:
**09.10.1996 Patentblatt 1996/41**

(73) Patentinhaber:
- **BOEHRINGER INGELHEIM INTERNATIONAL GmbH**
  **55218 Ingelheim am Rhein (DE)**
  Benannte Vertragsstaaten:
  **GB IE**
- **Boehringer Ingelheim Pharma KG**
  **55216 Ingelheim am Rhein (DE)**
  Benannte Vertragsstaaten:
  **BE CH DE DK ES FR GR IT LI LU MC NL PT SE AT**

(72) Erfinder:
- **ROOS, Otto**
  **D-55270 Schwabenheim (DE)**
- **LOESEL, Walter**
  **D-55435 Gau-Algesheim (DE)**
- **ARNDTS, Dietrich**
  **D-55437 Appenheim (DE)**

(74) Vertreter: **Laudien, Dieter, Dr.**
**Boehringer Ingelheim GmbH,**
**Binger Strasse 173**
**55216 Ingelheim (DE)**

(56) Entgegenhaltungen:
**EP-A- 0 037 934** **EP-A- 0 251 194**
**EP-A- 0 288 048** **EP-A- 0 358 957**
**DE-A- 3 023 717**

- **IND. J. CHEM., SECT. B, Bd. 20B, no.10, 1981 Seiten 885-890, V.K. KANSAL, A.P. BHADURI 'Possible Antihypertensive Agents: Syntheses of N-Aralkyl-beta-substituted Phenylethylamines & N-Alkyl/acyl-6,7-dimethoxy-1-((alpha-pheny l-beta-substituted phenyl)ethyl)-1,2,3,4-tetrahydroisoquinoli nes'**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 736 011 B1

## Beschreibung

Die Erfindung betrifft neue anellierte Dihydropyridinessigsäurederivate, Verfahren zu ihrer Herstellung und diese Verbindungen enthaltende Arzneimittel.

Aus der EP-A 37 934 sind Dihydroisochinoline bekannt geworden. Die dort genannten Verbindungen sind cardioton wirksam und weisen eine kontraktilitätssteigernde und blutdruckbeeinflussende Wirkkomponente auf. Sie sind zur Verbesserung der Gewebsdurchblutung und der Gewebssauerstoffversorgung vorgeschlagen worden. Diese Verwendungsmöglichkeiten beruhen auf der vaskulären Wirksamkeit der Verbindungen. In EP-A 251 194 und EP-A 288 048 ist beschrieben worden, daß carbocyclisch und heterocyclisch anellierte Dihydropyridine cardioprotektive beziehungsweise hirnprotektive Wirkung besitzen und einen völlig neuen Typ Ca-antagonistischer Verbindungen verkörpern. In WO 91/11010 ist die Verwendung solcher Verbindungen für hirnprotektive Mittel, für die Behandlung chronisch inflammatorischer Prozesse und zur Hemmung der Blutgerinnung bzw. Blutplättchenaggregation beschrieben.

Gegenstand der vorliegenden Erfindung sind neue carbocyclisch und heterocyclisch anellierte Dihydropyridine und die pharmazeutische Verwendung dieser Verbindungen. Die neuen Verbindungen haben wertvolle therapeutisch nutzbare Eigenschaften. Sie sind als cardioprotektive Mittel, als hirnprotektive Mittel (insbesondere bei der Behandlung von Patienten, die einen Schlaganfall erlitten haben oder gefährdet sind, einen Schlaganfall zu erleiden), als Mittel für die Behandlung chronisch inflammatorischer Prozesse (z.B. Bronchialasthma, Arthritis) verwendbar. Ferner können diese Verbindungen als Mittel mit antiproliferativer Wirkung und als Mittel zur Behandlung der Colitis Ulcerosa und des Morbus Crohn verwendet werden.

Die Erfindung betrifft Verbindungen der allgemeinen Formel I

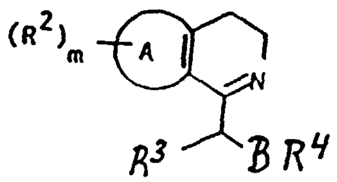

worin

A einen Benzo, Indolo oder Thienorest bedeutet; worin, wenn A Benzo ist, m 2 oder 3 ist, (vorzugsweise 2, wobei die beiden $R^2$ in den Positionen 6 und 7 sind) und die Substituenten $R^2$ unabhängig voneinander Hydroxy, $(C_1$-$C_4)$Alkoxy, Benzyloxy, Halogen (F, Cl, Br, J), $(C_1$-$C_4)$Alkyl, Methansulfonyloxy oder Methansulfonamido, oder zwei benachbarte Substituenten $R^2$ zusammen -O-$CH_2$-O oder -O-$CH_2$-$CH_2$-O- sein können; und wenn A Indolo oder Thieno ist, m Null ist;

B die Gruppe -O-, -S- oder -$CHR^5$ - bedeutet, worin $R^5$ Wasserstoff, $(C_1$-$C_6)$Alkyl, Phenyl oder Benzyl ist;

$R^3$ 2-oder 3-Thienyl, $(C_4$-$C_7)$Cycoalkyl, $(C_4$-$C_6)$Cycloalkyl$(C_1$-$C_5)$alkyl oder

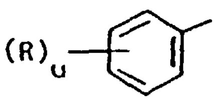

oder

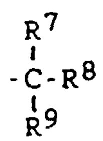

bedeutet, worin

R $(C_1$-$C_4)$-Alkyl, Hydroxy, -$N_3$, Halogen (F, Cl, Br, I), $CF_3$ oder $(C_1$-$C_4)$-Alkoxy ist,

u 0, 1, 2 oder 3 ist, und

$R^7$, $R^8$ und $R^9$ unabhängig voneinander Methyl, Ethyl, Propyl, Phenyl oder Benzyl sein können, wobei nicht mehr als 2 der Substituenten gleichzeitig Phenyl oder Benzyl sein können;

$R^4$

(a) verzweigtes oder unverzweigtes Alkenyl mit 3 bis 6 Kohlenstoffatomen, das durch Phenyl substituiert sein kann, ist oder

(b) verzweigtes oder unverzweigtes Alkinyl mit 3 bis 6 Kohlenstoffatomen, das durch Phenyl substituiert sein kann, ist
oder

(c) verzweigtes oder unverzweigtes Alkyl mit 1-13 Kohlenstoffatomen ist, wobei das Alkyl substituiert sein kann durch
Hydroxy,
$(C_1-C_4)$Alkoxy,
Di$(C_1-C_4)$Alkylamino,
Furyl,
Pyridyl,
Pyrrolidinyl, N-Methylpyrrolidinyl,
Morpholino,
Indolyl,
Nitrilo,
Thienyl,
Adamantyl,
Cyclohexyl,
Phenoxy,
Benzyloxy,
Naphthyloxy oder
Phenyl,
(wobei dieses Phenyl oder das in der Phenoxygruppe bzw. Benzyloxygruppe enthaltene Phenyl ein-, zwei- oder dreifach durch Hydroxy, $(C_1-C_4)$Alkoxy, Benzyloxy, Halogen (F, Cl, Br, J), $CF_3$, $N_3$, $NO_2$, $(C_1-C_4)$Alkyl, Adamantyl, $-SO_2NH_2$, $NHCOCH_3$, $-NHSO_2CH_3$ oder -C(O)O-$R_{14}$, [worin $R_{14}$ $(C_3-C_7)$Cycloalkyl oder verzweigtes oder unverzweigtes $(C_1-C_6)$Alkyl ist,
wobei das Alkyl durch Phenyl substituiert sein kann,
wobei dieses Phenyl durch Halogen (F, Cl, Br, J), $CF_3$, $C_1$- oder $C_2$-Alkyl, $C_1$- oder $C_2$-Alkoxy ein- bis 3-fach substituiert sein kann]
oder durch die Brücke $-O-CH_2-O-$ substituiert sein kann), oder
durch 2 unsubstituierte Phenylgruppen;

(d)

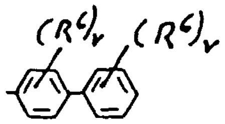

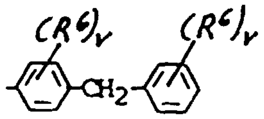

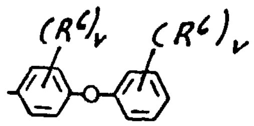

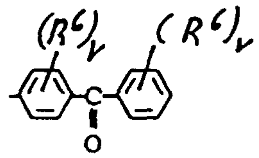

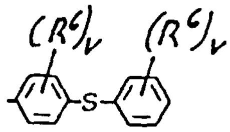

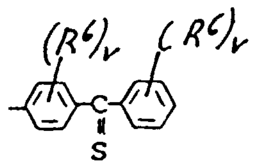

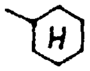

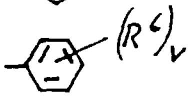

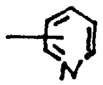

oder

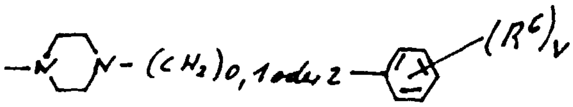

ist,
worin $R^6$ $(C_1\text{-}C_4)$Alkyl, Hydroxy, $-N_3$, Halogen (F, Cl, Br, J),

$CF_3$, $NO_2$ oder $(C_1\text{-}C_4)$Alkoxy ist und
v 0, 1, 2 oder 3 ist,
oder deren Salze mit physiologisch verträglichen Säuren oder Komplexbildern.

Verbindungen der Formel I bilden Tautomere der Formel II

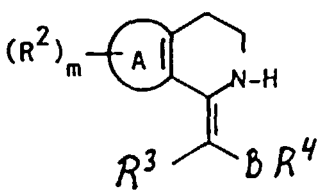

II

Die Tautomeren sind nach bekannten Methoden trennbar z.B. durch Säulenchromatographie oder selektive Reduktion ($NaBH_4$ beziehungsweise katalytische Reduktion).

Die Verbindungen der Formel II können in cis und/oder trans-Form vorliegen:

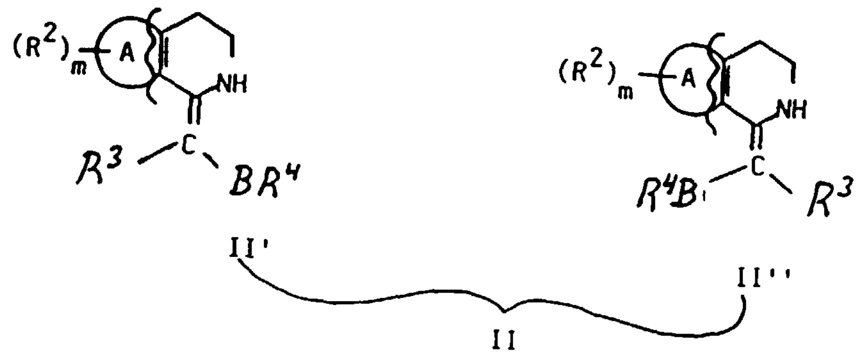

II'

II''

II

Wenn die Struktur einer Verbindung nicht ausdrücklich angegeben ist, ist die Angabe der Formel I so zu verstehen, daß Struktur II ebenfalls umfaßt wird.

In den im Text verwendeten Definitionen können die Reste und Gruppen jeweils gleich oder verschieden sein, d.h. wenn einer der zuvor genannten Substituenten in einem bestimmten Molekül mehrfach vorkommt, kann die jeweilige Bedeutung im Rahmen der Definitionsbreite frei gewählt werden.

Mit Alkyl sind insbesondere $C_1$-$C_6$-Alkyl- und $C_1$-$C_4$-Alkylradikale gemeint die ihrerseits substituiert sein können oder als Alkylradikale Bestandteil einer funktionellen Gruppe - wie Alkoxy oder Alkylthio - sind. Die Alkylradikale umfassen die Methyl-, Ethyl-, n-Propyl-, iso-Propyl-, n-Butyl-, sek-Butyl-, iso-Butyl-, tert-Butylradikale sowie die verschiedenen isomeren Pentyl- und Hexylradikale, wie etwa das Isopentyl, das Neopentyl, das n-Pentyl und das n-Hexylradikal.

Halogene sind Fluor, Chlor, Brom und Jod, vorzugsweise Fluor, Chlor und Brom und in zweiter Linie Jod.

$C_3$-$C_7$-Cycloalkyl sind Cyclopropan, Cyclobutan, Cyclopentan, Cyclohexan und Cycloheptan.

$C_3$-$C_6$-Alkine sind die isomeren Hexine, Pentine, Butine und Propine bevorzugt ist Propargyl

$C_3$-$C_6$-Alkene sind die isomeren Hexene, Pentene, Butene und Propene bevorzugt ist Allyl.

Ein bevorzugter Aspekt der Erfindung sind Verbindungen der allgemeinen Formel I worin

A Benzo bedeutet; m 2 ist, (wobei die beiden $R^2$ vorzugsweise in den Positionen 6 und 7 sind) und die Substituenten $R^2$ unabhängig voneinander Hydroxy, $(C_1$-$C_4)$Alkoxy, Benzyloxy,

Halogen (F, Cl, Br, J), oder $(C_1$-$C_4)$Alkyl, oder zwei benachbarte Substituenten $R^2$ zusammen $-O-CH_2-O$ sein können;

B die Gruppe -O-, -S- oder $-CHR^5$ - bedeutet, worin $R^5$ Wasserstoff, Methyl, Phenyl oder Benzyl ist;

$R^3$ 2- oder 3-Thienyl, $(C_4$-$C_7)$Cycoalkyl, oder

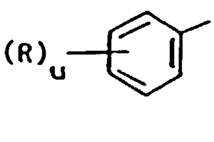

oder

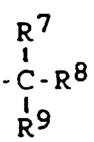

bedeutet, worin

R $(C_1$-$C_4)$-Alkyl, Halogen (F, Cl, Br, I), $CF_3$ oder $(C_1$-$C_4)$-Alkoxy ist,

u 0, 1, 2 oder 3 ist, und

$R^7$, $R^8$ und $R^9$ unabhängig voneinander Methyl, Ethyl, Propyl, Phenyl oder Benzyl sein können, wobei nicht mehr als 2 der Substituenten gleichzeitig Phenyl oder Benzyl sein können;

$R^4$

(a) verzweigtes oder unverzweigtes Alkenyl mit 3 bis 6 Kohlenstoffatomen, das durch Phenyl substituiert sein kann, ist oder

(b) verzweigtes oder unverzweigtes Alkinyl mit 3 bis 6 Kohlenstoffatomen, das durch Phenyl substituiert sein kann, ist
oder

(c) verzweigtes oder unverzweigtes Alkyl mit 1-13 Kohlenstoffatomen ist, wobei das Alkyl substituiert sein kann durch
Thienyl,
Adamantyl,
Cyclohexyl,
Phenoxy,
Benzyloxy,
oder
Phenyl,
(wobei dieses Phenyl oder das in der Phenoxygruppe bzw. Benzyloxygruppe enthaltene Phenyl ein-, zwei- oder dreifach durch $(C_1\text{-}C_4)$Alkoxy, Halogen (F, Cl, Br, J), $CF_3$, $N_3$, $NO_2$, $(C_1\text{-}C_4)$Alkyl, oder $-C(O)\text{-}O\text{-}(CH_2)_{1\text{-}3}\text{-}CH_3$
oder durch die Brücke $-O\text{-}CH_2\text{-}O-$ substituiert sein kann), oder
durch 2 unsubstituierte Phenylgruppen;

(d)

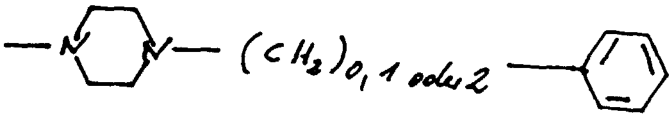

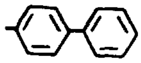

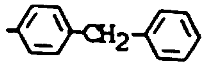

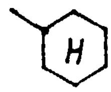

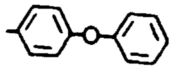

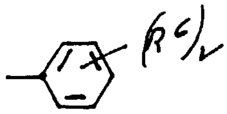

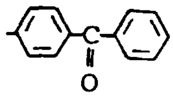

oder

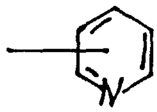

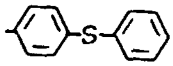

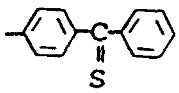

ist,
worin $R^6$ $(C_1-C_4)$Alkyl, Hydroxy, $-N_3$, Halogen (F, Cl, Br, J), $CF_3$, $NO_2$ oder $(C_1-C_4)$Alkoxy ist und

v 0, 1, 2 oder 3 ist,

oder deren Salze mit physiologisch verträglichen Säuren oder Komplexbildnern.

Von den Verbindungen I, worin A Benzo ist, sind solche bevorzugt, worin m 2 ist und die beiden $R^2$ unabhängig voneinander Methoxy, Hydroxy, Benzyloxy, Methyl oder Chlor oder zusammen $-OCH_20-$ sind, wobei die beiden $R^2$ in den Positionen 6 und 7 stehen, insbesondere solche Verbindungen, worin $R^2$ Methoxy, Hydroxy, Benzyloxy oder Methyl ist, insbesondere solche worin beide $R^2$ gleich sind und Hydroxy oder Methoxy bedeuten.
Hervorzuheben sind Verbindungen I, worin $R_4$ eine der folgenden Bedeutungen hat:

a) Vinyl, vorzugsweise durch Phenyl substituiert;

b) unsubstituiertes Alkyl mit 4 bis 9 Kohlenstoffatomen, das, falls es verzweigt ist, Methyl als Nebenkette(n) enthält;

c) substituiertes Alkyl mit 1 bis 4 (vorzugsweise 2 oder 3) Kohlenstoffatomen, das, falls es verzweigt ist, eine

Methylgruppe als Nebenkette enthält,
wobei das Alkyl substituiert sein kann durch ein oder 2 Phenylgruppen,
Phenoxy
Benzyloxy
Thienyl ( vorzugsweise 3-Thienyl)
Cyclohexyl oder
1- oder 2-Adamantyl,
wobei, wenn das Alkyl durch eine Phenylgruppe oder durch Phenoxy oder Benzyloxy substituiert ist, die betreffende Phenylgruppe substituiert sein kann durch
Methyl,
Methoxy (1 bis 3 Methoxygruppen),
Cl, Br, $CF_3$,
$NO_2$ oder
$C(O)O(CH_2)_3CH_3$

d)

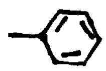

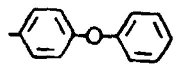

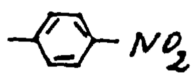

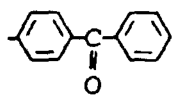

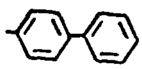

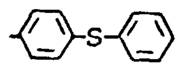

oder

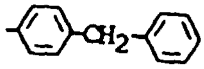

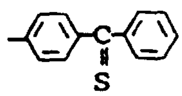

insbesondere solche, worin $R_4$ eine der folgenden Bedeutungen hat:

a) $R_4$ $(C_4\text{-}C_7)$Alkyl;
b) substituiertes Alkyl mit 1 bis 4 (vorzugsweise 2 oder 3) Kohlenstoffatomen, des, falls es verzweigt ist; eine Methylgruppe als Nebenkette enthält,
wobei das Alkyl substituiert sein kann durch ein oder 2 Phenylgruppen,
Phenoxy
Benzyloxy
Thienyl(vorzugsweise 3-Thienyl) oder
Cyclohexyl,
wobei, wenn das Alkyl durch Phenoxy oder Benzyloxy oder vorzugsweise eine Phenylgruppe substituiert ist, die betreffende Phenylgruppe substituiert sein kann durch Methoxy (1 bis 3 Methoxygruppen),
Cl, Br, $CF_3$,
$NO_2$ oder
$C(O)O(CH_2)_3CH_3$;

c)

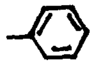

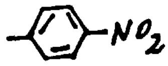

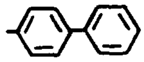

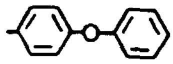

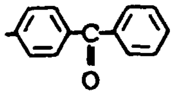

Ferner sind solche Verbindungen (I) hervorzuheben, worin B O, S $CH_2$ oder $C(CH_3)H$ ist, vorzugsweise solche, worin B O ist.

Ferner sind solche Verbindungen hervorzuheben, worin $R_3$ Phenyl (das 1-, 2- oder 3-fach durch Methoxy, Halogen oder $CF_3$ substituiert sein kann), Cyclohexyl, Thienyl oder tert. Butyl ist, insbesondere solche Verbindungen, worin $R_3$ Phenyl, Methoxyphenyl, Cl-Benzyl, Di-Cl-Benzyl, oder Cyclohexyl ist. Hervorzuheben sind ferner besonders solche Verbindungen, worin $R_3$ Thienyl (vorzugsweise 3-Thienyl) oder tert. Butyl ist. Ferner sind solche Verbindungen hervorzuheben, worin A Benzo ist, das in den Positionen 6 und 7 substituiert ist, worin die beiden Substituenten $R_2$ unabhängig voneinander Methoxy, Benzyloxy, Hydroxy, Methyl oder Chlor sind, insbesondere worin $R_2$ in Position 6 Methoxy, Hydroxy oder Benzyloxy ist und $R_2$ in Position 7 Methoxy oder Methyl ist, vorzugsweise worin A die 6,7-Dimethoxybenzogruppe ist.

Besonders hervorzuheben sind Verbindungen worin $R_4$ eine der folgenden Bedeutungen hat

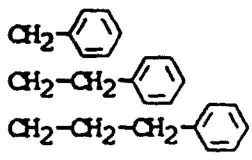

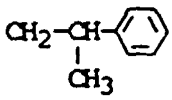

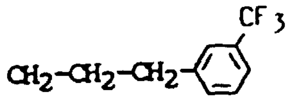

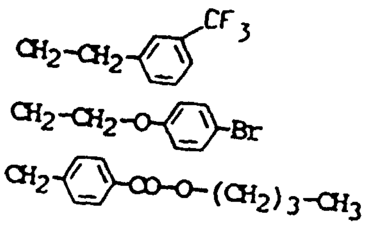

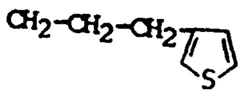

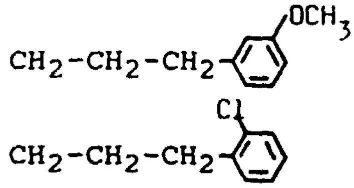

$(CH_2)_4-CH_3$

insbesondere solche, worin B O oder $CH_2$ ist und A wie oben definiert ist und vorzugsweise 6,7-Dimethoxybenzo ist. Innerhalb dieser Gruppe von Verbindungen sind solche bevorzugt, worin $R^3$ tert. Butyl, Thienyl oder vorzugsweise Phenyl oder Cyclohexyl ist.

Die Verbindungen der Formel I können nach an sich bekannten Verfahren hergestellt werden, vorzugsweise analog zu der in der deutschen Patentanmeldung P 37 18 570.5, EP 358 957, EP 37 934, EP 251 794 und EP 288 048 beschriebenen Methode.

In Gegenwart eines Kondensationsmittels kann eine Verbindung der allgemeinen Formel IV

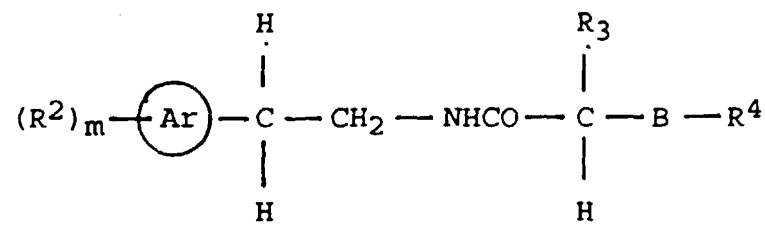

in der $R^2$, $R^3$, $R^4$ und m, wie oben definiert sind und Ar Phenyl, Indolyl oder 2- oder 3-Thienyl bedeutet, zu den entsprechenden Verbindungen cyclisiert werden.

Als Kondensationsmittel für dieses Verfahren eignen sich starke Lewis-Säuren, wie z.B. Phosphoroxychlorid, Phosphorpentachlorid, Phosphortrichlorid, Phosphorpentoxid, Titantetrachlorid, Bortrifluorid, Zinntetrachlorid, aber auch anorganische Säuren, wie z.B. Polyphosphorsäure, Schwefelsäure, Fluorsulfonsäure und Fluorwasserstoffsäure, oder Gemische von Kondensationsmitteln wie z.B. ein Gemisch von Phosphoroxychlorid und Phosphorpentachlorid, oder ein Gemisch von Phosphorpentoxid und ($C_1$-$C_4$) Alkylsulfonsäure z.B. mit einem $P_2O_5$-Anteil von ca. 10 Gewichtsprozenten.

Die Cyclisierung kann in Gegenwart oder Abwesenheit eines Lösungsmittels durchgeführt werden. Geeignet sind alle inerten Lösungsmittel, soweit sie eine ausreichende Löslichkeit für die Reaktionspartner besitzen und

einen ausreichend hohen Siedepunkt aufweisen, beispielsweise Benzol, Alkylbenzole (z.B. Toluol, Xylol), Chlorbenzole, Chloroform, Acetonitril, Dekalin. Eine bevorzugte Variante des Verfahrens besteht darin, als Kondensationsmittel Phosphoroxychlorid im Gemisch mit Acetonitril oder ein ($C_1$-$C_4$)Alkylsulfonsäure-/Phosphorpentoxid-Gemisch, ohne Zusatz von Lösungsmitteln zu verwenden.

Bevorzugt erfolgt die Cyclisierung mit Phosphoroxychlorid/Acetonitril oder in schwierigen Fällen mit einem Gemisch von Phosphorpentoxid und ($C_1$-$C_4$)Alkylsulfonsäure (bevorzugt Methansulfonsäure).
Die Umsetzung kann innerhalb eines großen Temperaturbereichs, vorzugsweise unter Erwärmen auf 50°C bis etwa den Siedepunkt des Reaktionsgemisches, durchgeführt werden.

Die erforderliche Reaktionsdauer liegt je nach Ausgangsverbindung IV zwischen 2 und 15 Stunden.

Ausgangsverbindungen:

Die Ether der allgemeinen Formel IV können nach den üblichen in der Literatur beschriebenen Verfahren erhalten werden. Bevorzugt ist die Verwendung der "Mesylat- und der Bromid-Methode", insbesondere der Mesylat-Methode. Zu beachten ist bei dem bevorzugten Verfahren, daß bei der Umsetzung auf den üblichen Basenzusatz verzichtet werden muß, um die Bildung von unerwünschten nichtetherischen Nebenprodukten zu vermeiden.

Die Thioether der allgemeinen Formel IV können nach den üblichen in der Literatur beschriebenen Verfahren erhalten werden. Bevorzugt ist die "Mesylat-Methode": Umsatz eines Mercaptans mit dem Mesylat des N-(2-(3,4-Dimethoxyphenyl)ethyl)-mandelsäureamids in lösungsmittelfreiem und basenfreiem Medium bei 100°C.

Die Alkane der allgemeine Formel IV werden erhalten durch Umsetzung der 2-Phenylalkylcarbonsäuren mit 2-(3,4-Dimethoxyphenylethylamin zu den ring offenen Amiden (bevorzugt mittels der Carbonyldiimidazol-Methode).

Die Verbindungen der Formel I sind Basen und können auf übliche Weise mit anorganischen oder organischen Säuren sowie Salz- und Komplexbildnern in beliebige physiologisch unbedenkliche Addukte (Salze) überführt werden.

Zur Salzbildung geeignete Säuren sind beispielsweise Salzsäure, Bromwasserstoffsäure, Jodwasserstoffsäure, Fluorwasserstoffsäure, Schwefelsäure, Phosphorsäure, Salpetersäure, Essigsäure, Propionsäure, Buttersäure, Capronsäure, Valeriansäure, Oxalsäure, Malonsäure, Bernsteinsäure, Maleinsäure, Fumarsäure, Milchsäure, Weinsäure, Zitronensäure, Apfelsäure, Benzoesäure, p-Hydroxybenzoesäure, Phthalsäure, Zimtsäure, Salicylsäure, Ascorbinsäure, Methansulfonsäure und dergleichen.

Die Verbindungen können oral, parenteral oder topisch verabreicht werden. Der gewünschte therapeutische Dosis ist von der Indikation und Darreichungsform abhängig und kann experimentell bestimmt werden. Geeignete Anwendungsformen sind beispielsweise Tabletten, Kapseln, Zäpfchen, Lösungen, Säfte, Emulsionen, Aerosole oder dispersible Pulver. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxypolymethylen, Carboxymethylcellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageehüllen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können.

Säfte der erfindungsgemäßen Wirkstoffe bzw. Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielweise Kondensationsprodukte von Fettalkoholen mit Ethylenoxid, oder Schutzstoffe, wie p-Hydroxybenzoate, enthalten.

Injektionslösungen werden in üblicher Weise, z.B. unter Zusatz von Konservierungsmitteln, wie p-Hydroxybenzoaten, oder Stabilisatoren, wie Alkalisalzen der Ethylendiamintetraessigsäure, hergestellt und in Injektionsflaschen oder Ampullen abgefüllt.

Die eine oder mehrere Wirkstoffe bzw. Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Geeignete Zäpfchen lassen sich beispielsweise durch Vermischen mit dafür vorgesehenen Trägermitteln, wie Neutralfetten oder Polyethylenglykol bzw. dessen Derivaten herstellen.

Die Verbindungen können sowohl enteral als auch parenteral verabreicht werden. Als Dosis für die orale

Anwendung werden 0,1 bis 500 mg Wirkstoff pro Dosis, für die i.v.-Anwendung von 0,05 bis 150 mg pro Dosis vorgeschlagen. Der gewünschte therapeutische Dosis ist von der Indikation und Darreichungsfonn abhängig und kann experimentell bestimmt werden.

Die Arzneimittel sind für orale oder parenterale, gegebenenfalls auch topische Verabreichung geeignet. Als Arzneimittelformen dienen vorwiegend Tabletten, Dragees, Ampullen und Saftzubereitungen. Die Einzeldosis zu diesen Arzneimittelformen beträgt zwischen 1,0 und 200 mg, vorzugsweise 20 und 50 mg pro 75 kg Körpergewicht. Je nach der Schwere des Falles sind täglich im allgemeinen 1 bis 3 Einzeldosen zu verabreichen.

Die folgenden Beispiele sollen die Erfindung näher erläutern:

Beispiel 1:

O-(3-Phenylpropyl)-phenylessigsäure

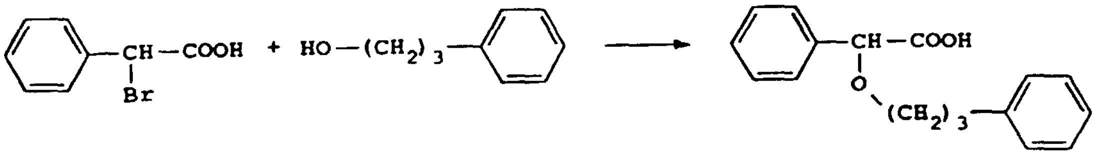

7,8 g (0,2 Mol) Kalium werden in 136 g (1 Mol) 3-Phenyl-1-propanol durch Erwärmen auf 100° gelöst und nach Abkühlung auf Z.T.. tropfenweise mit einer Lösung von 21,5 g (0,1 Mol) α-Bromphenylessigsäure in 20 ml 3-Phenyl-1-propanol versetzt.

Nach 2,5-stündigem Erhitzen auf 110°C werden bei Z.T. 500 ml Diethylether zugegeben und die Lösung 3 mal mit je 100 ml Wasser extrahiert. Die vereinigten Wasserextrakte werden unter Eiskühlung mit 30 ml 4N HCl angesäuert; das abgeschiedene Öl wird in Ether aufgenommen, mit Wasser gewaschen und über MgSO4 getrocknet. Nach Abdestillieren des Lösungsmittels verbleiben 23,4 g (86,6 % d.Th). des gewünschten Carbonsäureethers als öliger Rückstand, der ohne weitere Reinigung für die Folgeumsetzung verwendet werden kann.

N-[2-(3,4-Dimethoxyphenyl)ethyl]-0-(3-phenylpropyl)phenylessigsäureamid

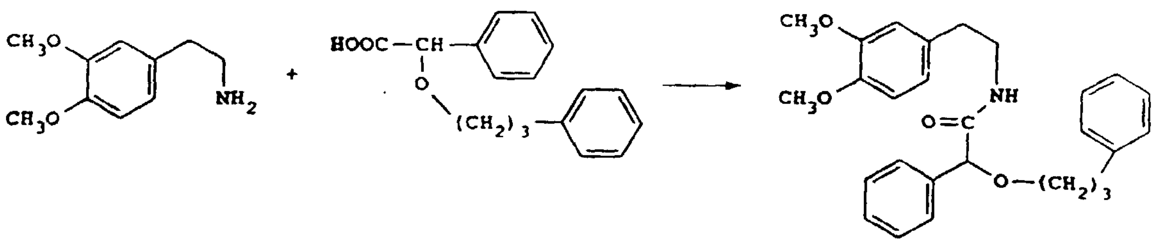

30,3 g (113 mmol) 0-(3-Phenylpropyl)-phenylessigsäure werden in 250 ml abs. Tetrahydrofuran gelöst, mit 18,14 g (112 mmol) Carbonyldiimidazol versetzt und 2 Stunden bei Raumtemperatur gerührt. Zu dieser Reaktionslösung werden unter Eiskühlung 20,27 g (112 mmol) 2-(3,4-Dimethoxyphenyl) ethylamin in 50 ml abs. Tetrahydrofuran zugetropft und 15 Stunden bei Raumtemperatur weitergerührt. Danach wird das Lösungsmittel im Vakuum abdestilliert, der Rückstand in 250 ml Essigester gelöst, 3 mal mit je 100 ml 1N HCl, 2 mal mit je 75 ml gesättigter $NaHCO_3$-Lösung und 2 mal mit je 50 ml gesättigter NaCl-Lösung gewaschen. Die Essigesterlösung wird über MgSO4 getrocknet und im Vakuum eingeengt.

Das ölige Reaktionsprodukt von 44,28 g (91,3 % d. Th.) kann ohne weitere Reinigung weiterverarbeitet werden.

Variante:

N-[2-(3,4-Dimethoxyphenyl)ethyl]-mandelsäureamid

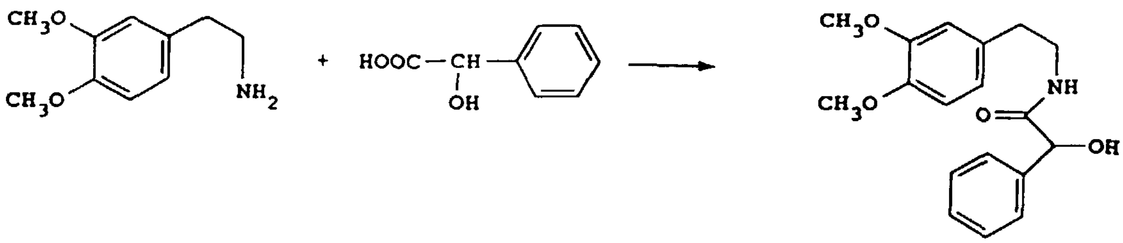

33,2 g (0,2 Mol) Mandelsäuremethylester und 72 g (0,4 Mol) 2-(3,4-Dimethoxyphenyl)-ethylamin werden unter $N_2$-Atmosphäre und Rühren 5 Stunden lang auf 160-165°C am absteigenden Kühler erhitzt (zur Entfernung des freiwerdenden Methanols). Der erkaltete Rückstand wird in 300 ml Essigester gelöst, 2 mal mit je 100 ml 2N HCl, 1 mal mit gesättigter $NaHCO_3$-Lösung und gesättigter NaCl-Lösung gewaschen, über MgSO4 getrocknet und vom Lösungsmittel abdestilliert.

Der kristalline Rückstand wird in 200 ml Essigester gelöst und das Reaktionsprodukt mit 160 ml n-Hexan ausgefällt.

Ausbeute: 51,2 g (80,95 % d. Th.), Fp: 89-91°.

N-[2-(3,4-Dimethoxyphenyl)ethyl]-0-methylmandelsäure-amid

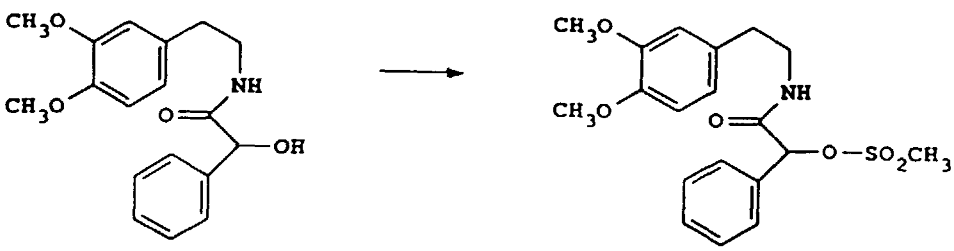

Zu einer Lösung von 19,6 g (62,2 mmol) N-[2-(3,4-Dimethoxyphenyl)ethyl-]mandelsäureamid in 60 ml abs. Pyridin werden unter Rühren und Kühlen 7,8 (69 mmol) Methansulfonsäurechlorid zugetropft, wobei die Reaktionslösung auf -15° bis -5° gehalten wird. Nach 5-stündigem Rühren bei Z.T. wird mit 200 g Eis versetzt und unter Eiskühlung mit 180 ml 4N HCl angesäuert. Die saure Lösung wird 2 mal mit je 400 ml Essigester ausgeschüttelt und die organische Phase 2 mal mit je 50 ml $H_2O$, 2 mal mit gesättigter NaCl-Lösung gewaschen, über MgSO4 getrocknet und das Lösungsmittel im Vakuum abdestilliert. Der kristalline Rückstand wird mit 100 ml Essigester und 50 ml n-Heptan umgefällt und dabei 16,3 g (66,6 % d Th.) des Reaktionsproduktes vom Fp. 89-90°C erhalten.

N-[2-(3,4-Dimethoxyphenyl)ethyl]-0-(3- phenylpropyl)phenylessigsäure-amid

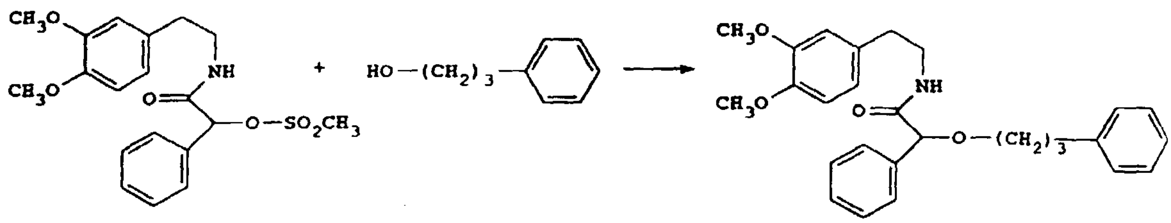

1,95 g (5 mmol) N-[2-(3,4-Dimethoxyphenyl)ethyl]-0-mesylmandelsäure-amid werden in 3,4 g (25 mmol) 3-Phenyl-1-propanol gelöst und 30 Min. unter Rühren auf 100°C erhitzt. Nach Abkühlung auf Z.T. wird das Reaktionsprodukt in 100 ml Essigester gelöst, 2 mal mit gesättigter $NaHCO_3$-Lösung und 1 mal mit NaCl-Lösung gewaschen, über MgSO4 getrocknet und das Lösungsmittel im Vakuum abdestilliert.

Das ölige Reaktionsprodukt wird über eine Kieselgel-Säule mit Essigester /n-Heptan 1:1 chromatographisch gereinigt und dabei 1.54 g (71,5 % d. Th.) des gewünschten Ethers als Öl erhalten.

(R,S)-(3,4-Dihydro-6,7-dimethoxyisochinolin-1-yl)-2-Phenyl-2-(3-phenylpropyl)-ether

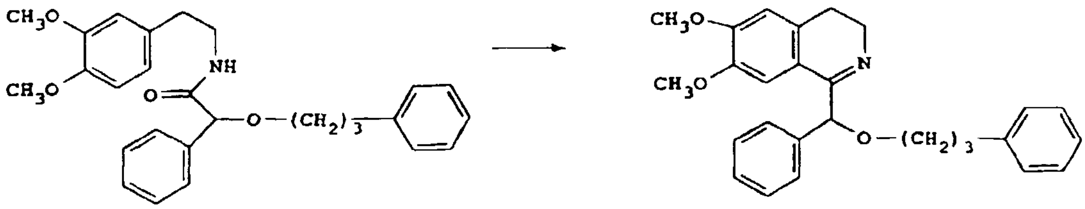

Eine Lösung von 44,28 g (102,3 mmol) N-[2-(3,4-Dimethylphenyl)ethyl]-0-(3-Phenylpropyl)phenylessigsäure-amid und 46,93 g (307 mmol)) Phosphoroxychlorid in 300 ml Acetonitril wird 1 Stunde in einer $N_2$-Atmosphäre unter Rückfluß zum Sieden erhitzt. Nach Abkühlung auf Z.T. wird das Reaktionsgemisch in 1 l Essigester gelöst, 2 mal mit je 150 ml Eiswasser, 3 mal mit je 150 ml gesättigter $NaHCO_3$-Lösung und 2 mal mit je 100 ml gesättigter NaCl-Lösung gewaschen, über $MgSO_4$ getrocknet und das Lösungsmittel im Vakuum abdestilliert. Der Rückstand wird in 110 ml Aceton gelöst und unter Rühren und Eiskühlung mit etherischer HCl angesäuert, wobei 17,6 g (38,68 % d. Th.) des gewünschten Reaktionsproduktes als Hydrochlorid auskristallisiert; Fp: 176-178°C

Beispiel 2:

N-[2-(3,4-Dimethoxyphenyl)ethyl]-S-(3-phenylpropyl)phenylessigsäure-amid

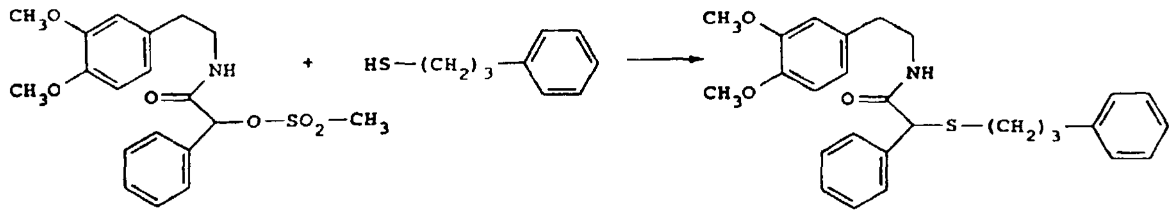

3,93 g (10 mmol) N-[2-(3,4-Dimethoxyphenyl)ethyl]-O-mesylmandelsäure-amid und 4,56 g (30 mmol) 3-Phenyl-n-propylmercaptan werden 30 Min. unter Rühren auf 100°C erhitzt.

Das erkaltete Reaktionsprodukt wird in 100 ml Essigester gelöst, 2 mal mit je 20 ml gesättigter $NaHCO_3$-Lösung und 1 mal mit gesättigter NaCl-Lösung gewaschen. Nach Trocknen über MgSO4 wird das Lösungsmittel im Vakuum abdestilliert. Der ölige Rückstand wird über eine Kieselgel-Säule mit Essigester/n-Heptan 1:1 als Eluat, chramatographisch gereinigt. Ausbeute: 2,55 g (56,8 % d. Th.)

(R,S)-(3,4-Dihydro-6,7-dimethoxyisochinolin-1-yl)-2-phenyl-2-(3-phenylpropyl)-thioether

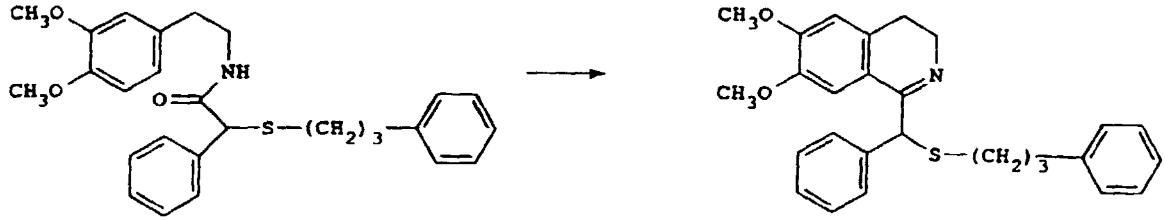

2,55 g (5,67 mmol) N-[2-(3,4-Dimethoxyphenyl)ethyl]-S-(3-phenylpropyl)phenylessigsäure-amid und 2,6 g (17 mmol) Phosphoroxychlorid werden in 35 ml Acetonitril 1,5 Stunden unter $N_2$-Atmosphäre und Rühren auf 100° erhitzt. Die Reaktionslösung wird bei Z.T. mit 125 ml Essigester versetzt., 3 mal mit je 25 ml Eiswasser, 3 mal mit je 25 ml gesättigter $NaHCO_3$-Lösung und 1 mal mit gesättigter NaCl-Lösung gewaschen und über MgSO4 getrocknet.

Nach Abdestillieren der organischen Phase im Vakuum wird der ölige Rückstand über eine Kieselgel-Säule mit Essigester als Eluat gereinigt und dabei 1,18 g des gewünschten Reaktionsproduktes amorph erhalten.

Beispiel 3:

N-[2-(3,4-Dimethoxyphenyl)ethyl]-2,5-diphenylpentancarbonsäure-amid

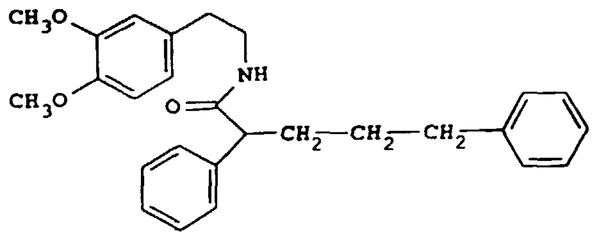

Eine Lösung von 3,0 g (11,8 mmol) 2,5-Diphenylpentancarbonsäure und 1,91 g (11,8 mmol) Carbonyldiimidazol wird 1 Stunde bei Raumtemperatur gerührt und anschließend mit einer Lösung von 2,13 g (11,8 mmol) 2-(3,4-Dimethylethoxphenyl)ethylamin in 20 ml Tetrahydrofuran versetzt, über Nacht wird bei R.T. weitergerührt und nach Abdestillieren des Lösungsmittels der Rückstand in Essigester gelöst. Die organische Phase wird 2 mal mit je 15 ml 1N HCl, 2 mal mit je 20 ml gesättigter $NaHCO_3$-Lösung und anschließend mit einer gesättigten NaCl-Lösung gewaschen. Nach Trocknen über MgSO4 und Abdestillieren des Lösungsmittels verbleiben 4,9 g (100% d. Th.) des erwünschten Endproduktes, das als Öl ohne weitere Reinigung weiterverarbeitet wird.

(R,S)-(3,4-Dihydro-6,7-dimethoxyisochinolin-1-yl)-1,4-diphenylbutan

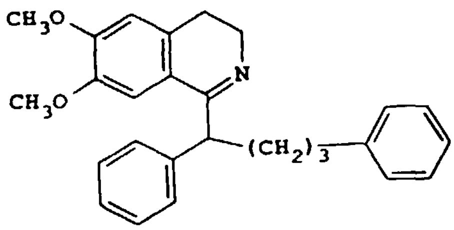

Eine Lösung von 4,9 g (11,75 mmol) N-[2-(3,4-Dimethoxyphenyl)ethyl]-2,5-diphenylpentancarbonsäure-amid und 539 g (35,2 mmol) Phosphoroxychlorid in 40 ml abs. Acetonitrii wird 1,5 Stunden unter $N_2$-Atmosphäre und Rühren zum Sieden am Rückfluß erhitzt. Die Reaktionslösung wird mit 150 ml Essigester verdünnt und nacheinander 2 mal mit je 40 ml Eiswasser, 3 mal mit je 30 ml gesättigter $NaHCO_3$-Lösung und mit gesättigter NaCl-Lösung gewaschen und die organische Phase über $MgSO_4$ getrocknet. Nach Abdestillieren des Lösungsmittels im Vakuum wird der ölige Rückstand in Aceton gelöst und mit etherischer Salzsäure angesäuert.

Das gewünschte Reaktionsprodukt fällt dabei als amorphe Festsubstanz in der HCl-Salzform an.

Ausbeute: 2,6 g (50,8 % d. Th.)

Die folgende Tabellen fassen Beispiele von erfindungsgemäßen Verbindungen zusammen. Die Herstellung dieser Verbindungen kann analog zu den oben beschriebenen Verfahren erfolgen.

Tabelle 1:

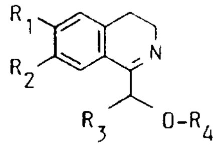

| Nr. | $R_1$ | $R_2$ | $R_3$ | $R_4$ | Fp. °C | Salzform |
|---|---|---|---|---|---|---|
| 1 | $OCH_3$ | $OCH_3$ | $C_6H_5$ | 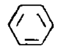 | amorph | OX |
| 2 | $OCH_3$ | $OCH_3$ | $C_6H_5$ | 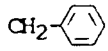 | 142-145 | OX |
| 3 | $OCH_3$ | $OCH_3$ | $C_6H_5$ | 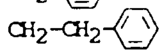 | 165-168 | CL |
| 4 | $OCH_3$ | $OCH_3$ | $C_6H_5$ | 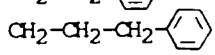 | 176-178 | CL |
| 5 | $OCH_3$ | $OCH_3$ | $C_6H_5$ | 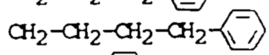 | 57-60 | OX |
| 6 | $OCH_3$ | $OCH_3$ | $C_6H_5$ | 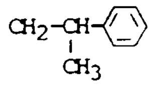 | 112-114 | OX |
| 7 | $OCH_3$ | $OCH_3$ | $C_6H_5$ | 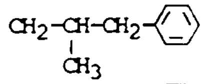 | | |
| 8 | $OCH_3$ | $OCH_3$ | $C_6H_5$ | 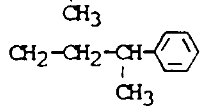 | | |
| 9 | $OCH_3$ | $OCH_3$ | $C_6H_5$ | 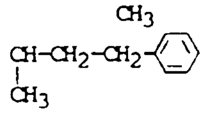 | | |
| 10 | $OCH_3$ | $OCH_3$ | $C_6H_5$ | 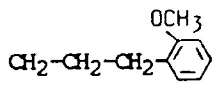 | 123-126 | CL |
| 11 | $OCH_3$ | $OCH_3$ | $C_6H_5$ | 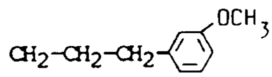 | 70-80 | OX |
| 12 | $OCH_3$ | $OCH_3$ | $C_6H_5$ | 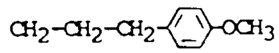 | amorph | CL |
| 13 | $OCH_3$ | $OCH_3$ | $C_6H_5$ | 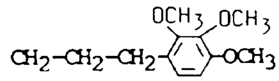 | amorph | OX |
| 14 | $OCH_3$ | $OCH_3$ | 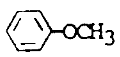 | 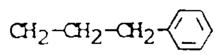 | amorph | OX |
| 15 | $OCH_3$ | $OCH_3$ | 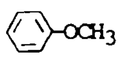 | 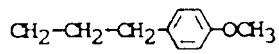 | amorph | OX |
| 16 | $OCH_3$ | $OCH_3$ | $C_6H_5$ | 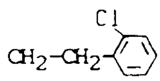 | | |
| 17 | $OCH_3$ | $OCH_3$ | $C_6H_5$ | 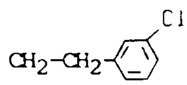 | | |
| 18 | $OCH_3$ | $OCH_3$ | $C_6H_5$ | 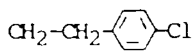 | 141-143 | OX |
| 19 | $OCH_3$ | $OCH_3$ | $C_6H_5$ | 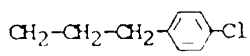 | 143-145 | MA |
| 20 | $OCH_3$ | $OCH_3$ | $C_6H_5$ | 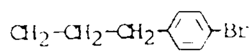 | 75-80 | OX |

| Nr. | $R_1$ | $R_2$ | $R_3$ | $R_4$ | Fp. °C | Salzform |
|---|---|---|---|---|---|---|
| 21 | $OCH_3$ | $OCH_3$ | 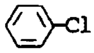 | 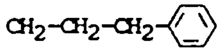 | 139-142 | OX |
| 22 | $OCH_3$ | $OCH_3$ | 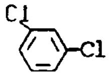 | 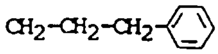 | 159-163 | OX |
| 23 | $OCH_3$ | $OCH_3$ | $C_6H_5$ | 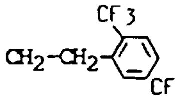 | | |
| 24 | $OCH_3$ | $OCH_3$ | $C_6H_5$ | 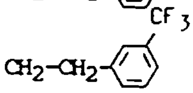 | 108-110 | CL |
| 25 | $OCH_3$ | $OCH_3$ | $C_6H_5$ | 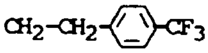 | | |
| 26 | $OCH_3$ | $OCH_3$ | $C_6H_5$ | 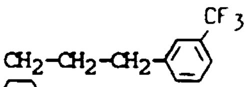 | amorph | OX |
| 27 | $OCH_3$ | $OCH_3$ | $C_6H_5$ | 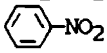 | 138-140 | OX |
| 28 | $OCH_3$ | $OCH_3$ | $C_6H_5$ | 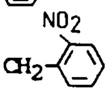 | | |
| 29 | $OCH_3$ | $OCH_3$ | $C_6H_5$ | 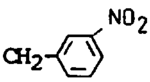 | 157-159 | MA |
| 30 | $OCH_3$ | $OCH_3$ | $C_6H_5$ | 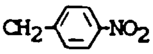 | | |
| 31 | $OCH_3$ | $OCH_3$ | $C_6H_5$ | 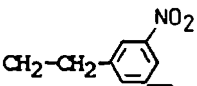 | 180-182 | CL |
| 32 | $OCH_3$ | $OCH_3$ | $C_6H_5$ | 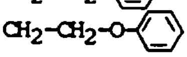 | amorph | CL |
| 33 | $OCH_3$ | $OCH_3$ | $C_6H_5$ | 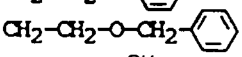 | amorph | OX |
| 34 | $OCH_3$ | $OCH_3$ | $C_6H_5$ | 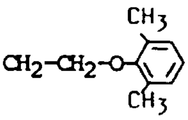 | | |
| 35 | $OCH_3$ | $OCH_3$ | $C_6H_5$ | 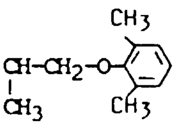 | | |
| 36 | $OCH_3$ | $OCH_3$ | $C_6H_5$ | 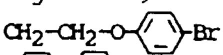 | 120-122 | MA |
| 37 | $OCH_3$ | $OCH_3$ | $C_6H_5$ | 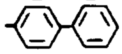 | 80-100 (Zersetzung) | OX |
| 38 | $OCH_3$ | $OCH_3$ | $C_6H_5$ | 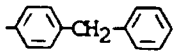 | | |
| 39 | $OCH_3$ | $OCH_3$ | $C_6H_5$ | 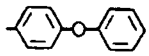 | 50-75 (Zersetzung) | OX |
| 40 | $OCH_3$ | $OCH_3$ | $C_6H_5$ | 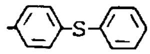 | | |
| 41 | $OCH_3$ | $OCH_3$ | $C_6H_5$ | 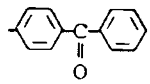 | 60-80 (Zersetzung) | OX |

| Nr. | $R_1$ | $R_2$ | $R_3$ | $R_4$ | Fp. °C | Salzform |
|---|---|---|---|---|---|---|
| 42 | $OCH_3$ | $OCH_3$ | $C_6H_5$ | 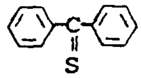 | | |
| 43 | $OCH_3$ | $OCH_3$ | $C_6H_5$ | 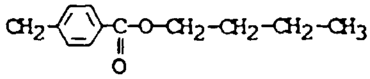 | amorph | OX |
| 44 | $OCH_3$ | $OCH_3$ | $C_6H_5$ | 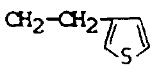 | amorph | OX |
| 45 | $OCH_3$ | $OCH_3$ | $C_6H_5$ | 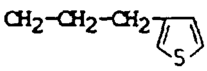 | 60-70 (Zersetzung) | OX |
| 46 | $OCH_3$ | $OCH_3$ | $C_6H_5$ | 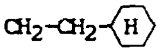 | 124-127 | OX |
| 47 | $OCH_3$ | $OCH_3$ | $C_6H_5$ | 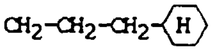 | 145-147 | OX |
| 48 | $OCH_3$ | $OCH_3$ | 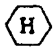 | 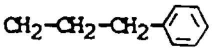 | 138-140 | OX |
| 50 | OH | $OCH_3$ | $C_6H_5$ | 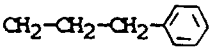 | 65-80 (Zersetzung) | OX |
| 51 | 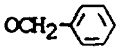 | $OCH_3$ | $C_6H_5$ | 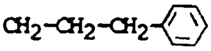 | amorph | OX |
| 52 | OH | $CH_3$ | $C_6H_5$ | 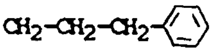 | 75-120 (Zersetzung) | OX |
| 53 | 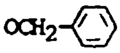 | $CH_3$ | $C_6H_5$ | 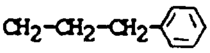 | amorph | OX |
| 54 | OH | Cl | $C_6H_5$ | 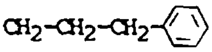 | | |
| 55 | 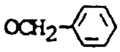 | Cl | $C_6H_5$ | 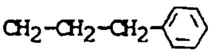 | | |
| 56 | OH | Cl | 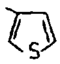 | 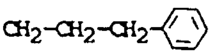 | | |
| 57 | $OCH_3$ | $OCH_3$ | 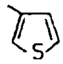 | 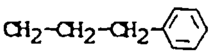 | | |
| 58 | $OCH_3$ | $OCH_3$ | $C_6H_5$ | 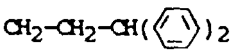 | amorph | CL |
| 59 | $OCH_3$ | $OCH_3$ | $C_6H_5$ | $CH_2-CH_2-CH_2-CH_3$ | | |
| 60 | $OCH_3$ | $OCH_3$ | $C_6H_5$ | $CH_2-CH_2-CH_2-CH_2-CH_3$ | 105-110 | OX |
| 61 | $OCH_3$ | $OCH_3$ | $C_6H_5$ | $CH_2-CH_2-CH_2-CH_2-CH_2-CH_3$ | 82-85 | OX |

| Nr. | $R_1$ | $R_2$ | $R_3$ | $R_4$ | Fp. °C | Salzform |
|---|---|---|---|---|---|---|
| 62 | $OCH_3$ | $OCH_3$ | $C_6H_5$ | $CH_2-CH_2-CH_2-CH_2-CH_2-CH_2-CH_3$ | 96-100 | OX |
| 63 | $OCH_3$ | $OCH_3$ | $C_6H_5$ | $CH_2-CH_2-CH_2-CH_2-CH_2-CH_2-CH_2-CH_3$ | 60-65 | OX |
| 64 | $OCH_3$ | $OCH_3$ | $C_6H_5$ | $CH_2-CH-CH_2-CH_2-CH_3$<br>$CH_3$ | ~60 (Zers.) | OX |
| 65 | $OCH_3$ | $OCH_3$ | $C_6H_5$ | $CH_2-CH_2-CH-CH_2-CH_3$<br>$CH_3$ | | |
| 66 | $OCH_3$ | $OCH_3$ | $C_6H_5$ | $CH_2-CH_2-CH_2-CH-CH_3$<br>$CH_3$ | | |
| 67 | $OCH_3$ | $OCH_3$ | $C_6H_5$ | $CH_3$<br>$CH_2-C-CH_3$<br>$CH_3$ | 150-152 | OX |
| 68 | $OCH_3$ | $OCH_3$ | $C_6H_5$ | $CH_3$<br>$CH_2-CH_2-C-CH_3$<br>$CH_3$ | 105-110 | OX |
| 69 | $OCH_3$ | $OCH_3$ | $C_6H_5$ | $CH_3$<br>$CH_2-CH_2-CH_2-C-CH_3$<br>$CH_3$ | | |
| 70 | $OCH_3$ | $OCH_3$ | $C_6H_5$ | $CH_2-CH_2$-1-Adamantan | 155-158 | OX |
| 71 | $OCH_3$ | $OCH_3$ | $C_6H_5$ | $CH_2-CH_2$-2-Adamantan | | |
| 72 | $OCH_3$ | $OCH_3$ | $C_6H_5$ | $CH_2-CH_2-CH_2$-1-Adamantan | | |
| 73 | $OCH_3$ | $OCH_3$ | $C_6H_5$ | $CH_2-CH_2-CH_2$-2-Adamantan | | |
| 74a | $OCH_3$ | $OCH_3$ | $C_6H_5$ | $(CH_2)_3$- 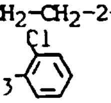 | 135-136 | OX |

Tabelle 2:

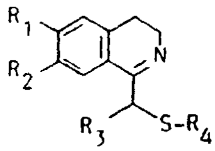

| Nr. | $R_1$ | $R_2$ | $R_3$ | $R_4$ | Fp. °C | Salzform |
|---|---|---|---|---|---|---|
| 74 | $OCH_3$ | $OCH_3$ | $C_6H_5$ | $CH_2-CH_2-CH_2-$ 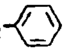 | | |
| 75 | $OCH_3$ | $OCH_3$ | $C_6H_5$ | $CH_2-CH-$ 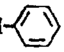<br>$CH_3$ | | |
| 76 | $OCH_3$ | $OCH_3$ | $C_6H_5$ | $CH_2-CH_2-$ 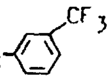 | | |
| 77 | $OCH_3$ | $OCH_3$ | $C_6H_5$ | $CH_2-CH_2-O-$ 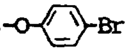 -Br | | |
| 78 | $OCH_3$ | $OCH_3$ | $C_6H_5$ | $CH_2-$ 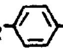 $-CO-O-(CH_2)_3-CH_3$ | | |
| 79 | OH | $OCH_3$ | $C_6H_5$ | $CH_2-CH_2-CH_2-$ 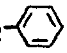 | | |
| 80 | OH | $CH_3$ | 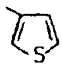 | $CH_2-CH_2-CH_2-$ 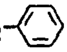 | | |
| 81 | OH | $CH_3$ | 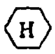 | $CH_2-CH_2-CH_2-$ 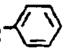 | | |
| 82 | $OCH_3$ | $OCH_3$ | $C_6H_5$ | $CH_2-CH_2-CH_2-$ 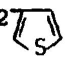 | | |
| 83 | $OCH_3$ | $OCH_3$ | 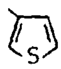 | $CH_2-CH_2-CH_2-$ 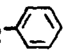 | | |
| 84 | OH | Cl | 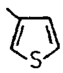 | $CH_2-CH_2-CH_2-$ 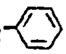 | | |
| 85 | OH | Cl | 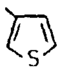 | $CH_2-CH_2-CH_2-$ 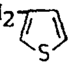 | | |
| 86 | $OCH_3$ | $OCH_3$ | 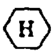 | $CH_2-CH_2-CH_2-$ 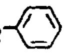 | | |
| 87 | OH | $CH_3$ | $C_6H_5$ | $CH_2-CH_2-CH_2-$ 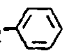 | | |

Tabelle 3:

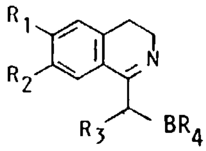

| Nr. | $R_1$ | $R_2$ | $R_3$ | B $R_4$ | Fp. °C | Salzform |
|---|---|---|---|---|---|---|
| 88 | $OCH_3$ | $OCH_3$ | $C_6H_5$ | $CH_2-CH_2-$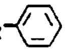 | 190-192 | CL |
| 89 | $OCH_3$ | $OCH_3$ | $C_6H_5$ | $CH_2-CH_2-CH_2-$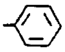 | amorph | CL |
| 90 | $OCH_3$ | $OCH_3$ | $C_6H_5$ | $CH_2-CH_2-CH_2-CH_2-$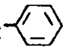 | 185-195 | Cl |
| 91 | $OCH_3$ | $OCH_3$ | $C_6H_5$ | $CH_2-CH(CH_3)-$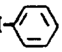 | | |
| 92 | $OCH_3$ | $OCH_3$ | $C_6H_5$ | $CH_2-CH_2-CH(CH_3)-$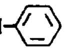 | | |
| 93 | $OCH_3$ | $OCH_3$ | $C_6H_5$ | $CH(CH_3)-CH_2-CH_2-$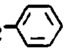 | | |
| 94 | $OCH_3$ | $OCH_3$ | $C_6H_5$ | $CH_2-CH_2-CH_2-$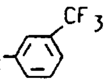 | | |
| 95 | $OCH_3$ | $OCH_3$ | $C_6H_5$ | $CH_2-CH_2-CH_2-O-$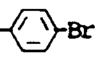-Br | | |
| 96 | $OCH_3$ | $OCH_3$ | $C_6H_5$ | $CH_2-CH_2-$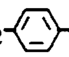$-CO-O-(CH_2)_3-CH_3$ | | |
| 97 | $OCH_3$ | $OCH_3$ | $C_6H_5$ | $-CH_2-CH_2-CH_2-$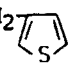 | | |
| 98 | $OCH_3$ | $OCH_3$ | 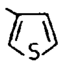 | $-CH_2-CH_2-CH_2-CH_2-$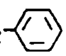 | | |
| 99 | $OCH_3$ | $OCH_3$ | 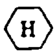 | $-CH_2-CH_2-CH_2-CH_2-$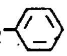 | | |
| 100 | $OCH_3$ | $OCH_3$ | 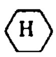 | $-CH_2-CH_2-CH_2-$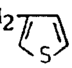 | | |
| 101 | $OCH_3$ | $OCH_3$ | t-Butyl | $-CH_2-CH_2-CH_2-CH_2-$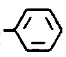 | | |
| 102 | $OCH_3$ | $OCH_3$ | t-Butyl | $-CH_2-CH_2-CH_2-$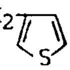 | | |

| Nr. | $R_1$ | $R_2$ | $R_3$ | B $R_4$ | Fp. °C | Salzform |
|---|---|---|---|---|---|---|
| 103 | OH | $OCH_3$ | $C_6H_5$ | $CH_2$-$CH_2$-$CH_2$-$CH_2$- 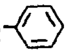 | | |
| 104 | OH | $OCH_3$ | 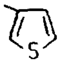 | $CH_2$-$CH_2$-$CH_2$-$CH_2$- 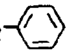 | | |
| 105 | OH | $CH_3$ | $C_6H_5$ | $CH_2$-$CH_2$-$CH_2$- 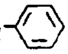 | | |
| 106 | OH | $CH_3$ | 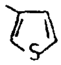 | $CH_2$-$CH_2$-$CH_2$- 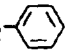 | | |
| 107 | OH | $CH_3$ | 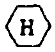 | $CH_2$-$CH_2$-CH- 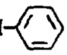 | | |
| 108 | OH | $CH_3$ | $C_6H_5$ | $CH_2$-$CH_2$-$CH_2$ 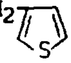 | | |
| 109 | OH | Cl | $C_6H_5$ | $CH_2$-$CH_2$-$CH_2$- 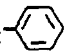 | | |
| 110 | OH | Cl | 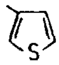 | $CH_2$-$CH_2$-$CH_2$- 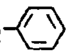 | | |
| 111 | OH | Cl | 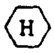 | $CH_2$-$CH_2$-$CH_2$- 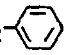 | | |
| 112 | OH | Cl | $C_6H_5$ | $CH_2$-$CH_2$-$CH_2$ 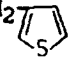 | | |
| 113 | $OCH_3$ | $OCH_3$ | t-Butyl | $CH_2$-$CH_2$-$CH_2$- 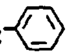 | | |
| 114 | $OCH_3$ | $OCH_3$ | t-Butyl | $CH_2$-$CH_2$-$CH_2$ 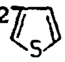 | | |
| 115 | $OCH_3$ | $OCH_3$ | 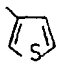 | $CH_2$-$CH_2$-$CH_2$ 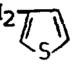 | | |
| 116 | OH | $OCH_3$ | 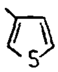 | $CH_2$-$CH_2$-$CH_2$ 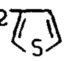 | | |
| 117 | $OCH_3$ | $OCH_3$ | $C_6H_5$ | $CH_2$-CH =CH- 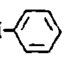 | amorph | Cl |
| 118 | $OCH_3$ | $OCH_3$ | $C_6H_5$ | $CH_2$-$CH_2$-$CH_2$-$CH_2$-$CH_3$ | 105-110 | OX |
| 119 | $OCH_3$ | $OCH_3$ | $C_6H_5$ | $CH_2$-$CH_2$-$CH_2$-$CH_2$-$CH_2$-$CH_3$ | 90-93 | OX |
| 120 | $OCH_3$ | $OCH_3$ | $C_6H_5$ | $CH_2$-$(CH_2)_5$-$CH_3$ | 96-100 | |
| 121 | $OCH_3$ | $OCH_3$ | $C_6H_5$ | $CH_2$-$(CH_2)_6$-$CH_3$ | amorph | OX |
| 122 | $OCH_3$ | $OCH_3$ | $C_6H_5$ | $CH_2$-$(CH_2)_8$-$CH_3$ | 45-53 Zers. | OX |
| 123 | $OCH_3$ | $OCH_3$ | $C_6H_5$ | $CH_2$-$CH_2$-$CH_2$-1-Adamantan | | |
| 124 | $OCH_3$ | $OCH_3$ | $C_6H_5$ | $CH_2$-$CH_2$-1-Adamantan | 156-159 | OX |

| Nr. | $R_1$ | $R_2$ | $R_3$ | B $R_4$ | Fp. °C | Salzform |
|---|---|---|---|---|---|---|
| 125 | $OCH_3$ | $OCH_3$ | $C_6H_5$ | $CH_2-(CH_2)_{10}-CH_3$ | amorph | OX |
| 126 | $OCH_3$ | $OCH_3$ | $C_6H_5$ | $CH_2-CH-CH_3$<br>$CH_3$ | 178-180 | CL |
| 127 | $OCH_3$ | $OCH_3$ | $C_6H_5$ | 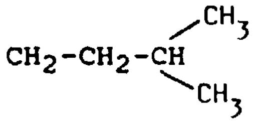 | 198-202 | Cl |
| 128 | $OCH_3$ | $OCH_3$ | $C_6H_5$ | $CH_2-CH_2-CH_3$ | 144-147 | OX |
| 129 | $OCH_3$ | $OCH_3$ | 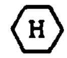 | $CH_2-CH_2-$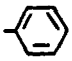 | 121-124 | OX |

Gegenstand der vorliegenden Erfindung ist ferner die Verwendung dieser neuen Verbindungen.

Die Verbindungen sind zur Behandlung von degenerativen und nekrotisierenden Erkrankungen des Gehirns vorteilhaft. Ebenso ist die vorbeugende Behandlung von durch solche Krankheiten gefährdeten Patienten möglich. Diese Wirkung der Verbindungen beruht nicht auf einer Verbesserung der Gewebsdurchblutung. Die Verbindungen sind somit für eine neuartige Behandlung von Epilepsie und der Alzheimer-Krankheit geeignet, und insbesondere bei der Behandlung von Patienten, die einen Schlaganfall erlitten haben oder gefährdet sind, einen Schlaganfall zu erleiden.

Ein weiterer Gegenstand der vorliegenden Erfindung ist die Verwendung der genannten Verbindungen für die Herstellung von Mitteln zur Behandlung chronisch inflammatorischer Prozesse, der Colitis Ulcerosa und des Morbus Crohn und von Mitteln mit antiproliferativer Wirkung. Die Wirkung der Verbindungen kann durch ihre Hemmung der unselektiven Kationenkanäle (UKK) erklärt werden.

Der Pathophysiologie des chronischen Bronchialasthma liegen entzündliche Prozesse zugrunde, die durch die Aktivierung inflammatorischer Zellen vermittelt werden. (BARNES, 1987; SEIFERT und SCHULTZ, 1991).

Die rezeptor-regulierte Aktivierung inflammatorischer Zellen (z.B. neutrophile Granulozyten und Mastzellen bzw. deren permanente Zellinien HL-60 Zellen oder sensibilisierte, d.h. Gammaglobulin E beladene RBL-Zellen) wird unabhängig von der Art der stimulierenden Agonisten

(z.B. Endothelin, PAF, Leukotriene, chemotaktisches Peptid fMLP oder Antigen gegen sensibilisierte Mastzellen) durch Blocker unselektiver Kationenkanäle (UKK) inhibiert (RINK, 1990). Durch diese Kanäle gelangt extrazelluläres Calcium in die Zellen, das für die Persistenz der rezeptor-vermittelten Zellaktivierungen erforderlich ist (PUTNEY, 1990). Wird diese Calciumzufuhr unterbrochen resultiert eine Blockade der Aktivierung inflammatorischer Zellen.

Klassische Calciumantagonisten vom Dihydropyridin- bzw. Phenylalkylamin-Typ hemmen weder UKKs noch inflammatorische Prozesse (WELLS et al., 1986).

Als Maß der Zellaktivierung bzw. als Maß von deren Hemmung durch UKK-Blocker wird fluorometrisch die Kinetik der cytoplasmatischen Calciumionenkonzentration in Fura-2-beladenen Zellen anhand der von GRYNKIEWICZ et al. (1985) beschriebenen Methode quantifiziert. Diese Vorgehensweise hat sich im Rahmen dieser Erfindung als zuverlässige Screeningmethode zur Auffindung von UKK-Blockern erwiesen.

Zur spezifischen Charakterisierung von Blockern der unselektiven Kationenkanäle eignet sich die sogenannte funktionelle THAPSIGARGIN-Inhibition. THAPSIGARGIN ist ein von THASTRUP et al. (Proc. Natl. Acad. Sci. (USA), 87, 2466-2470, 1990) beschriebener Tumorpromotor, der selektiv und irreversibel die $Ca^{2+}$ATPase intrazellulärer, $IP_3$-sensitiver $Ca^{2+}$-Speicher hemmt. Infolge dessen kommt es zu einer raschen Entleerung der $Ca^{2+}$-Speicher. Wie von J. PUTNEY (Calcium, 11, 611-624, 1990) beschrieben stellt die Entleerung dieser Speicher den physiologischen Reiz für die

Öffnung unselektiver Kationenkanäle in der Zellmembran dar. Die Folge ist ein massiver Einstrom von $Na^+$ und $Ca^{2+}$ in

die Zelle. Aufgrund dieser Eigenschaften eignet sich Thapsigargin als indirekter Stimulator zur agonisten- und $IP_3$-unabhängigen Öffnung unselektiver Kationenkanäle.

Im Rahmen der vorliegenden Erfindung wurde die Thapsigargin-Stimulation unselektiver Kationenkanäle an HL 60 Zellen (menschliche Leukämiezelle), an hippocampalen und corticalen Neuronenzellen sowie an RBL-Zellen (rat basophilic lymphoma cells) erfolgreich durchgeführt und somit wurde die Existenz dieser Kanäle in den jeweiligen Zellinien nachgewiesen.

Die zytoplasmatische $Ca^{2+}$Konzentration ($[Ca^{2+}]_i$) spielt eine wichtige Rolle bei der Zellproliferation und beim Tumorwachstum (Übersicht siehe L.R. ZACHARSKI, Journal of Medicine 19: 145-177, 1988). Insbesondere der durch Rezeptoraktivierung mit konsekutiver Inositoltrisphosphat-($IP_3$-)-Vermittlung stimulierte $Ca^{2+}$-Einstrom in die Zelle soll von entscheidender Bedeutung sein für die oncogene Zellproliferation (U. KIKKAWA und Y. NISHIZUKA, Ann. REV. CELL. BIOL. 2: 149-178, 1986). Dieser Mechanismus spielt auch eine Rolle bei der Metastasenbildung und der "Multi-Drug-Resistance". (Übersicht siehe die obengenannten Veröffentlichung von L.R. ZACHARSKI.) J. MED. 19: 145-177, 1980.

Diese Hypothese wird dadurch gestützt, daß Thapsigargin als indirekter Stimulator der unselektiven Kationenkanäle (UKK) sowohl zu einem $Ca^{2+}$-overload in der Zelle führt als auch ein hochwirksamer Tumorpromotor ist.(V. THASTRUP et al. Proceedings of the NATL. Acad. Sci: (USA) 87: 2466-2470, 1990.)

Die Blockade des $Ca^{2+}$-Einstroms durch die UKK führt zu einer Normalisierung der intrazellulären Ca-Ionenkonzentration und somit zu einer Hemmung des Tumorwachstums etc.

Klassiche Calciumantagonisten hemmen diese UKK nicht. Überraschend ist festgestellt worden, daß die Verbindungen dieser Erfindung den Calciumeinstrom in die Zelle durch die UKK hemmen.

Wie von S. H. MURCH et al. (Lancet 339 : 381-385, 15. Febr. 1992) gezeigt wurde, spielt Endothelin I eine wichtige pathophysiologische Rolle bei entzündlichen Darmerkrankungen wie der Colitis ulcerosa und des Morbus Crohn. Mit Hilfe immunhistochemischer Methoden konnte festgestellt werden, daß Patienten mit M. Crohn im Bereich der Submucosa und Patienten mit Colitis ulcerosa im Bereich der Lamina propria des Dickdarmepithels signifikant und stark erhöhte Endothelin I Konzentrationen im Vergleich zu gesunden Normalpersonen aufweisen. Es wird angenommen, daß die lokale Ausschüttung von Endothelin massive Vasospasmen mit konsekutiver disseminierter Ischämie mit Mikroinfarkten hervorruft, die als eigentliche Ursache der genannten Erkrankungen angesehen werden. Die vasospasmogene Effektivität des Endothelins wird über einen $Ca^{2+}$-overload vaskulärer Myozyten erklärt. Dabei löst Endothelin primär eine $IP_3$-vermittelte intrazelluläre $Ca^{2+}$-Freisetzung aus, an die sich ein massiver transmembranärer $Ca^{2+}$-Einstrom durch Dihydropyridin-insensitive Kanäle anschließt. (M. S. Simonson et al. Clin. Invest. Med. 14: 499-507, 1991; T. Masakai, J. Cardiovasc. Pharmacol. 13:Suppl. 5, S1-S4, 1989; D. W. Hay, R. J. Pharmacol. 100: 383-392, 1990) Bei diesen Kanälen handelt es sich um unselektive Kationen Kanäle, deren Existenz kürzlich auch in Zellen der Dickdarmmukosa beschrieben wurde. (Chr. Siemer und H. Gögelein, Europ. J. Physiol. 420: 319-328, 1992).

Als geeignetes Screening Modell zur Auffindung funktioneller Endothelinantagonisten hat sich die Endothelin stimulierte Aktivierung Fura-2 beladener menschlicher Leukämiezellen (HL 60 Zelle) bewährt. In Anlehnung an G. GRYNKIEWICZ et al. (J. Biol. Chem. 260:340-3450, 1985)läßt sich die intrazelluläre $Ca^{2+}$-Konzentration im Cytoplasma von HL 60 Zellen (Suspensionen) spektrofluorometrisch verfolgen und als Maß der Zellaktivierung durch Endothelin quantifizieren. Die Stimulation erfolgte durch Zusatz von 0.1 mM Endothelin, sie ließ sich dosisabhängig durch die erfindungsgemäßen Substanzen inhibieren.

Der funktionelle Endothelinantagonismus der erfindungsgemäßen Substanzen wird über eine Blockade unselektiver Kationen Kanäle vermittelt. Deshalb eignet sich auch der Nachweis eines funktionellen Thapsigargin-Antagonismus an RBL-hm1-Zellen als Screening Methode für funktionelle Endothelinantagonisten.

Ausführung der Untersuchung:

Für Screeningzwecke werden in $Ca^{2+}$ freiem Inkubationsmedium Fura-2-beladene adhäsive RBL-hm 1-Zellen mit 0,1 μM Thapsigargin stimuliert. Nach 4 Minuten wird extrazelluläres $Ca^{2+}$ auf 1,5 mM restituiert und anhand der Fura-2-Fluoreszenz der exzessive Anstieg der cytoplasmatischen $Ca^{2+}$-Konzentration infolge eines massiven transmembranären $Ca^{2+}$-Einstroms durch unselektive Kationenkanäle registriert.

Dieser Einstrom ist ausschließlich und dosisabhängig zu inhibieren durch unselektive Kationen-Kanal- Blokker. Weder klassische Kalziumantagonisten noch spezifische Blocker von Agonisten, die den $IP_3$- Turnover stimulieren können den durch Thapsigargin indirekt ausgelösten transmembranären $Ca^{2+}$-Einstrom hemmen. Die Verbindungen der vorliegenden Erfindung zeichnen sich durch eine Hemmung der UKK aus.

Die fluorometrische Calciummessung im Cytoplasma einzelner adhärenter RBL-hm1-Zellen erfolgt in Analogie zu der von KUDO und OGURA (1986) für neuronale Zellen beschriebenen Methode. Verwendet wird ein AXIOVERT 35 Fluoreszenzmikroskop von ZEISS in Kombination mit einem Imaging-System von HAMAMATSU, bestehend aus dem ICMS-Bildverarbeitungssystem, Restlichtkamera mit Kontrolleinheit und Bildverstärker DVS 3000.

Die Kinetik der cytoplasmatischen $Ca^{+2}$-Konzentration wird als Konzentrations-Zeit-Kurve nach der durch

Thapsigargin (0,1 µM) stimulierten Zellaktivierung fortlaufend aufgezeichnet. Verglichen werden die Kurven zweier aktivierter Zellkulturen in Gegenwart und Abwesenheit von 10 µM Testsubstanz. Die Fläche unter diesen Kurven (area under the curve = AUC) wird integriert und als Maß der Zellaktivierung registriert. Die inhibitorische Wirkungsstärke der getesteten UKK-Blocker wird ermittelt anhand folgender Beziehung:

$$\%H = 100 - \frac{AUC_{inh} \times 100}{AUC_{(Kontrolle)}}$$

%H = die prozentuale Hemmung des Calciumeinstroms durch unselektive Kationenkanäle der stimuliert und durch 10 µM Testsubstanz inhibiert wird.

$AUC_{inh}$ = Fläche unter der Kurve, die in Gegenwart des Stimulans plus 10 µM inhibitorischer Testsubstanz aufgezeichnet wird.

AUC Kontr. = Fläche unter der Kurve, die nur nach Zusatz des Stimulans registriert wird.

Literatur zu den obigen Erläuterungen:

BARNES P.J., I.W. RODGER und N.C. THOMSON
Pathogenesis of asthma, in "ASTHMA, basic mechanisms and clinical management"
ED by P.J. BARNES; ACADEMIC PRESS, LONDON, 1988

GRYNKIEWICZ G., M. POENIE und R.Y. TSIEN
A new generation of $Ca^{2+}$-indicators with greatly improved fluorescence properties
J. BIOL. CHEM. 260: 3440-3450, 1985

HIDE, M. und M.A. BEAVEN
Calcium influx in a rat mast cell (RBL-2H3) line
J. BIOL. CHEM. 266 15221-15229, 1991

KUDO, Y. und A. OGURA
Glutamate-induced inerease in intracellular $Ca^{2+}$-concentration in isolated hippocampal neurones
BR. J. PHARMACOL. 89: 191-198, 1986

PUTNEY, J.W., jr.
Capacitative Calcium entry revised
CELL CALCIUM 11: 611-624, 1990

RINK, T.J.
Receptor-mediated calcium entry
FEBS LETT. 268: 381-385, 1990

SEIFERT, R. und G. SCHULTZ
The superoxide forming NADPH oxidase of phagocytes: An enzym system regulated by multiple mechanism
REV. PHYSIOL. BIOCHEM. PHARMACOL., Vol. 117,
SPRINGER VERL., 1991

WELLS, E., C.G. JACKSON, S.T. HARPER, J. MANN and R.P. EAOY Characterization of primate bronchoalveolar mast cells II, inhibition of histamine, $LTC_4$ and $PGF_{2a}$ release from primate bronchoalveolar mast cells and a comparison with rat peritoneal mast cells
J. IMMUNOL. 137: 3941-3945, 1986.

Meßergebnisse:
Angegeben wird die prozentuale Hemmung der UKK nach Thapsigarginstimulation (0,1 µM Thapsigargin) in RBL-hm 1 Zellen. Die einheitliche Konzentration der Testsubstanzen ist $10^{-5}$ Mol).

Anhand des folgenden Tests kann die funktionelle antiinflammatorische Effektivität gezeigt werden:

Verwendet werden einzelne an Glasplättchen adhärente RBL-2H3-Zellen (eine den Mastzellen verwandte Tumorzellinie).

Die Kultivierung und Anhaftung der RBL-2H3-Zellen erfolgt in der HIDE und BEAVEN (1991) beschriebenen Methode. Zur Sensibilisierung der adhäsiven RBL-2H3-Zellen werden die Zellen 2 Stunden bei Raumtemperatur mit einer 1:2000 verdünnten käuflichen Gammaglobulin E-Lösung gegen einen Dinitrophenol-Rinderserumalbumin-Komplex (DNP-BSA-Antigen) inkubiert. Anschließend werden die Zellen gewaschen. Durch Zusatz von 0,1 ml DNP-BSA-Lösung (10 µg/ml) erfolgt eine massive immunologische Zellaktivierung, die durch einen cytoplasmatischen $Ca^{2+}$-overload vermittelt wird. Die fluorometrische Calciummessung im Cytoplasma einzelner adhärenter RBL-2H3-Zellen erfolgt in Analogie zu der von KUDO und OGURA (1986) für neuronale Zellen beschriebenen Methode, die auch weiter oben in dieser Beschreibung erläutert wird.

Als Vergleichssubstanz dient in diesen Untersuchungen (10 µM) Chromoglycat, das eine ca. 50 %ige Hemmung der antigen-induzierten Zellaktivierung bewirkt.

In diesem Test zeigen die oben genannten Verbindungen %H Werte, die den oben angegebenen Werten vergleichbar sind.

In Untersuchungen an Mikrokulturen verschiedener menschlicher Tumorzellinien mit Hilfe des Tetrazolium Assays zur Feststellung des antiproliferativen Effektes der erfindungsgemäßen Substanzen zeigte sich überraschenderweise, daß die geprüfte Verbindung 5 bis 100 mal wirkungsetärker als die Vergleichssubstanz Verapamil sind.

Die antiproliferative Effektivität der Testsubstanzen wurde mit Hilfe des von MOSMANN (J. IMMUNOL. METH. 65: 55-63, 1983), DENIZOT et al. (J. IMMUNOL. METH. 89: 271-277, 1986) und von J. ELIASON et al. (INT. J. CANCER 46: 113-117, 1990) beschriebenen MTT-Tests bestimmt. (MTT = [3-(4,5-dimethylthiazol-2yl)2,5-diphenyltetrazoliumbromid] von CHEMICON Inc. El Segundo, Ca, USA). Dieser Indikator wird nur von lebenden Zellen mit intakten Mitochondrien zu einem blauen Formazan Produkt metabolisiert. Folgende menschliche Tumorzellinien wurden in unserem Test verwendet: A 549 (Adenocarcinom der Lunge), A 431 (Epidermiscarcinom der Vulva), PC 3 (Adenocarcinom der Prostata), SK BR 3 (Adenocarcinom der Mamma), HT 29 (CX1 1) (Adenocarcinom des Colon) und K 562 (chronisch-myeloische Leukämiezelle).

Der Test wurde auf Mikrotiterplatten durchgeführt. Jedes Well enthielt 100 ml einer Zellsuspension (0,2 x $10^6$ Zellen/ml). Als Inkubationsmedium diente RPMI 1640 mit 10% Hitze-inaktiviertem fetalen Kälberserum und 50 µg/ml Gentamycin. Die Zellsuspensionen wurden 0, 24, 48 oder 72 Stunden bei gesättigter Luftfeuchtigkeit in einem $CO_2$ (5%) - Luft (95%)-Gemisch bei 37°C inkubiert in Gegenwart und Abwesenheit von variablen Konzentrationen antiproliferativer Testsubstanzen. Die Testsubstanzen wurden in DMSO (Endverdünnung: 0,1 %) gelöst. Anschließend wurden 10 µl MTT-Lösung (3 mg/ml) und nach 3 Stunden 100 ml einer 0,08 N HCl enthaltenden Isopropanollösung zugesetzt. Nach einer weiteren Stunde wurde die Lichtabsorption bei 570 nm (Vergleichswellenlänge 630 nm) in einem Mikroplattenleser ermittelt. Die Lichtabsorption ist direkt proportional zur Anzahl lebender Zellen. Die halbmaximalen Hemmkonzentrationen der untersuchten Substanzen lagen bei 1 µg/ml.

Die vasospasmolytische Effektivität der obengenannten funktionellen Endothelin- bzw. Thapsigargin-Antagonisten wurde am isolierten Gefäßpräparat bestätigt: An retrograd perfundierten spontan schlagenden LANGENDORFF Herzen aus Ratten wurde die coronare Perfusion fortlaufend mittels elektromagnetischer Flußmessung (Apparatur von Hugo Sachs Elektronik, MARCH), quantifiziert. Mit dieser Meßanordnung lassen sich Ausmaß, Dauer und Verlaufsform von Gefäßspasmen mit großer Genauigkeit registrieren. Perfundiert man mit 100 nM Endothelinkonzentration, so wird der coronare Perfusionsfluß von 11 auf 5 ml/min reduziert. Die Perfusionseinschränkung kann durch die erfindungsgemäßen Substanzen aufgehoben werden. Die Wirkungsstärken der erfindungsgemäßen Verbindungen bezüglich der Thapsigargininhibition an Fura-2- beladenen RBL-hm1-Zellen bzw. der Effektivität der Endothelininhibition an Fura-2 beladenen HL 60 Zellen korrelierte eindeutig mit der am Langendorffpräparat nachgewiesenen vasospasmolytischen Effektivität der untersuchten Substanzen. Es kann daraus geschlossen werden, daß dem vasospasmolytischen Endothelinantagonismus der untersuchten Substanzen eine Blockade unselektiver Kationen Kanäle unterliegt.

Pharmazeutische Anwendungsbeispiele

| a) Dragees | |
|---|---|
| 1 Drageekern enthält: | |

(fortgesetzt)

| a) Dragees | |
|---|---|
| Wirkstoff der allgemeinen Formel I | 30,0 mg |
| Milchzucker | 100,0 mg |
| Maisstärke | 75,0 mg |
| Gelatine | 3,0 mg |
| Magnesiumstearat | 2,0 mg |
| | 210,0 mg |

Herstellung

Die Mischung der Wirksubstanz mit Milchzucker und Maisstärke wird mit einer 10%-igen wässerigen Gelatinelösung durch ein Sieb mit 1 mm Maschenweite granuliert, bei 40°C getrocknet und nochmals durch ein Sieb gerieben. Das so erhaltene Granulat wird mit Magnesiumstearat gemischt und verpreßt. Die so erhaltenen Kerne werden in üblicher Weise mit einer Hülle überzogen, die mit Hilfe einer wässerigen Suspension von Zucker, Titandioxyd, Talkum und Gummi arabicum aufgebracht wird. Die fertigen Dragees werden mit Bienenwachs poliert.

| b) Tabletten | |
|---|---|
| Wirkstoff der allgemeinen Formel I | 30,0 mg |
| Milchzucker | 100,0 mg |
| Maisstärke | 70,0 mg |
| löslich Stärke | 7,0 mg |
| Magnesiumstearat | 3,0 mg |
| | 210,0 mg |

Herstellung

Wirkstoff und Magnesiumstearat werden mit einer wässerigen Lösung der löslichen Stärke granuliert, das Granulat getrocknet und innig mit Milchzucker und Maisstärke vermischt. Das Gemisch wird sodann zu Tabletten von 210 mg Gewicht verpreßt.

| c) Kapseln | |
|---|---|
| Wirkstoff gemäß Formel I | 20,0 mg |
| Milchzucker | 230,0 mg |
| Maisstärke | 40,0 mg |
| Talk | 10,0 mg |
| | 300,0 mg |

Herstellung

Wirkstoff, Milchzucker und Maisstärke werden zunächst in einem Mischer und dann in einer Zerkleinerungsmaschine vermengt. Das Gemisch wird nochmals in den Mischer gegeben, gründlich mit dem Talk vermengt und

maschinell in Hartgelatinekapseln abgefüllt.

| d) Tabletten | | |
|---|---|---|
| Wirkstoff gemäß Erfindung | | 40,0 mg |
| Milchzucker | | 100,0 mg |
| Maisstärke | | 50,0 mg |
| kolloidale Kieselsäure | | 2,0 mg |
| Magnesiumstearat | | 3,0 mg |
| | insgesamt | 200,0 mg |

Herstellung:

Der Wirkstoff wird mit einem Teil der Hilfsstoffe gemischt und mit einer Lösung der löslichen Stärke in Wasser granuliert. Nach dem Trocknen des Granulate wird der Rest der Hilfsstoffe zugemischt und die Mischung zu Tabletten verpreßt.

| e) Dragées | | |
|---|---|---|
| Wirkstoff gemäß Erfindung | | 20,0 mg |
| Milchzucker | | 100,0 mg |
| Maisstärke | | 65,0 mg |
| kolloidale Kieselsäure | | 2,0 mg |
| lösliche Stärke | | 5,0 mg |
| Megnesiumstearat | | 3,0 mg |
| | insgesamt | 195,0 mg |

Herstellung:

Der Wirkstoff und die Hilfsstoffe werden, wie in Beispiel 1 beschrieben, zu Tablettenkernen verpreßt, die mit Zucker, Talcum und Gummi arabicum in üblicher Weise dragiert werden.

| f) Suppositorien | |
|---|---|
| Wirkstoff gemäß Erfindung | 50,0 mg |
| Milchzucker | 250,0 mg |
| Suppositorienmasse q.s. ad | 1,7 g |

Herstellung:

Der Wirkstoff und der Milchzucker werden miteinander vermischt und die Mischung in der geschmolzenen
Suppositorienmasse gleichmäßig suspendiert. Die Suspensionen werden in gekühlte Formen zu Suppositorien von 1,7 g Gewicht ausgegossen.

| g) Ampullen | |
|---|---|
| Wirkstoff gemäß Erfindung | 20,0 mg |
| Natriumchlorid | 5,0 mg |
| Bi-destilliertes Wasser q.s. ad | 2,0 ml |

Herstellung:

Der Wirkstoff und das Natriumchlorid werden in bi-destilliertem Wasser gelöst und die Lösung in Ampullen steril abgefüllt.

| h) Ampullen | |
|---|---|
| Wirkstoff gemäß Erfindung | 10,0 mg |
| Natriumchlorid | 7,0 mg |
| Bi-destilliertes Wasser q.s. ad | 1,0 mg |

| i) Tropfen | |
|---|---|
| Wirkstoff gemäß Erfindung | 0,70 g |
| p-Hydroxybenzoesäuremethylester | 0,07 g |
| p-Hydroxybenzoesäurepropylester | 0,03 g |
| Entmineralisiertes Wasser q.s. ad | 100,00 ml |

Herstellung:

Der Wirkstoff und die Konservierungsmittel werden in entmineralsiertem Wasser gelöst, die Lösung filtriert und in Flaschen zu je 100 ml abgefüllt.

**Patentansprüche**

**1.** Verbindung der allgemeinen Formel I

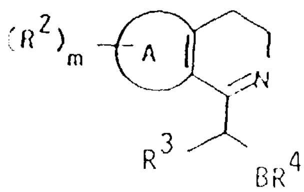

worin

A einen Benzo, Indolo oder Thienorest bedeutet; worin, wenn A Benzo ist, m 2 oder 3 ist, und die Substituenten

$R^2$ unabhängig voneinander Hydroxy, $(C_1-C_4)$Alkoxy, Benzyloxy, Halogen (F, Cl, Br, J), $(C_1-C_4)$Alkyl, Methansulfonyloxy oder Methansulfonamido, oder zwei benachbarte Substituenten $R^2$ zusammen $-O-CH_2-O$ oder $-O-CH_2-CH_2-O-$ sein können; und wenn A Indolo oder Thieno ist, m Null ist;

B die Gruppe -O-, -S- oder $-CHR^5$ - bedeutet, worin $R^5$ Wasserstoff, $(C_1-C_6)$Alkyl, Phenyl oder Benzyl ist;

$R^3$ 2-oder 3-Thienyl, $(C_4-C_7)$Cycoalkyl, $(C_4-C_6)$Cycloalkyl$(C_1-C_5)$alkyl oder

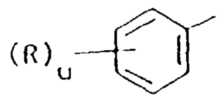

oder

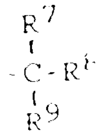

bedeutet, worin

R $(C_1-C_4)$-Alkyl, Hydroxy, $-N_3$, Halogen (F, Cl, Br, I), $CF_3$ oder $(C_1-C_4)$-Alkoxy ist,

u 0, 1, 2 oder 3 ist, und

$R^7$, $R^8$ und $R^9$ unabhängig voneinander Methyl, Ethyl, Propyl, Phenyl oder Benzyl sein können, wobei nicht mehr als 2 der Substituenten gleichzeitig Phenyl oder Benzyl sein können;

$R^4$

(a) verzweigtes oder unverzweigtes Alkenyl mit 3 bis 6 Kohlenstoffatomen, das durch Phenyl substituiert sein kann, ist oder

(b) verzweigtes oder unverzweigtes Alkinyl mit 3 bis 6 Kohlenstoffatomen, das durch Phenyl substituiert sein kann, ist
oder

(c) verzweigtes oder unverzweigtes Alkyl mit 1-13 Kohlenstoffatomen ist, wobei das Alkyl substituiert sein kann durch
Hydroxy,
$(C_1-C_4)$Alkoxy,
Di$(C_1-C_4)$Alkylamino,
Furyl,
Pyridyl,
Pyrrolidinyl, N-Methylpyrrolidinyl,
Morpholino,
Indolyl,
Nitrilo,
Thienyl,
Adamantyl,
Cyclohexyl,
Phenoxy,
Benzyloxy,
Naphthyloxy oder
Phenyl,
(wobei dieses Phenyl oder das in der Phenoxygruppe bzw. Benzyloxygruppe enthaltene Phenyl ein-, zwei- oder dreifach durch Hydroxy, $(C_1-C_4)$Alkoxy, Benzyloxy, Halogen (F, Cl, Br, J), $CF_3$, $N_3$, $NO_2$, $(C_1-C_4)$Alkyl, Adamantyl, $-SO_2NH_2$, $NHCOCH_3$, $-NHSO_2CH_3$ oder $-C(O)O-R_{14}$, [worin $R_{14}$ $(C_3-C_7)$Cycloalkyl oder verzweigtes oder unverzweigtes $(C_1-C_6)$Alkyl ist,
wobei das Alkyl durch Phenyl substituiert sein kann,
wobei dieses Phenyl durch Halogen (F, Cl, Br, J), $CF_3$, $C_1$- oder $C_2$-Alkyl, $C_1$- oder $C_2$-Alkoxy ein- bis 3-fach substituiert sein kann]
oder durch die Brücke $-O-CH_2-O-$ substituiert sein kann), oder
durch 2 unsubstituierte Phenylgruppen;

(d)

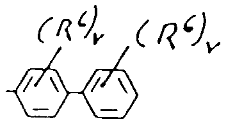

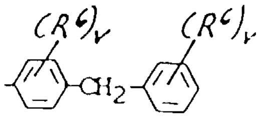

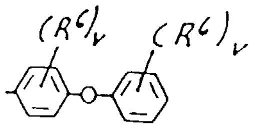

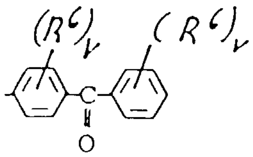

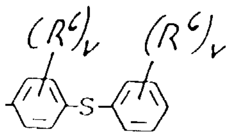

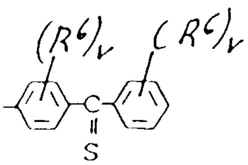

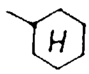

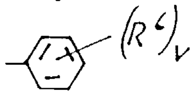

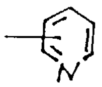

oder

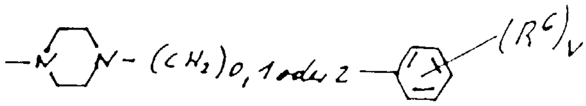

ist,
worin $R^6$ $(C_1-C_4)$Alkyl, Hydroxy, $-N_3$, Halogen (F, Cl, Br, J), $CF_3$, $NO_2$ oder $(C_1-C_4)$Alkoxy ist und

v 0, 1, 2 oder 3 ist,

oder deren Salze mit physiologisch verträglichen Säuren oder Komplexbildern.

**2.** Verbindung nach Anspruch 1, worin, wenn A Benzo ist, m 2 ist und die beiden $R^2$ in den Positionen 6 und 7 sind.

**3.** Verbindung nach Anspruch 1 oder 2, worin

A Benzo bedeutet; m 2 ist, und die Substituenten $R^2$ unabhängig voneinander Hydroxy, $(C_1-C_4)$Alkoxy, Benzyloxy, Halogen (F, Cl, Br, J), oder $(C_1-C_4)$Alkyl, oder zwei benachbarte Substituenten $R^2$ zusammen $-O-CH_2-O$ sein können;

B die Gruppe -O-, -S- oder $-CHR^5$ - bedeutet, worin $R^5$ Wasserstoff, Methyl, Phenyl oder Benzyl ist;

$R^3$ 2- oder 3-Thienyl, $(C_4$-$C_7)$Cycoalkyl, oder

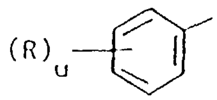

oder

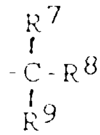

bedeutet, worin

R $(C_1$-$C_4)$-Alkyl, Halogen (F, Cl, Br, I), $CF_3$ oder $(C_1$-$C_4)$-Alkoxy ist,

u 0, 1, 2 oder 3 ist, und

$R^7$, $R^8$ und $R^9$ unabhängig voneinander Methyl, Ethyl, Propyl, Phenyl oder Benzyl sein können, wobei nicht mehr als 2 der Substituenten gleichzeitig Phenyl oder Benzyl sein können;

$R^4$

(a) verzweigtes oder unverzweigtes Alkenyl mit 3 bis 6 Kohlenstoffatomen, das durch Phenyl substituiert sein kann, ist oder
(b) verzweigtes oder unverzweigtes Alkinyl mit 3 bis 6 Kohlenstoffatomen, das durch Phenyl substituiert sein kann, ist
oder
(c) verzweigtes oder unverzweigtes Alkyl mit 1-13 Kohlenstoffatomen ist, wobei das Alkyl substituiert sein kann durch
Thienyl,
Adamantyl,
Cyclohexyl,
Phenoxy,
Benzyloxy,
oder
Phenyl,
(wobei dieses Phenyl oder das in der Phenoxygruppe bzw. Benzyloxygruppe enthaltene Phenyl ein-, zwei- oder dreifach durch $(C_1$-$C_4)$Alkoxy, Halogen (F, Cl, Br, J), $CF_3$, $N_3$, $NO_2$, $(C_1$-$C_4)$Alkyl, oder -C(O)-O-$(CH_2)_{1\text{-}3}$-$CH_3$
oder durch die Brücke -O-$CH_2$-O- substituiert sein kann), oder
durch 2 unsubstituierte Phenylgruppen;

(d)

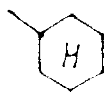

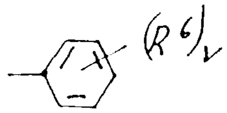

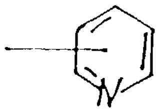

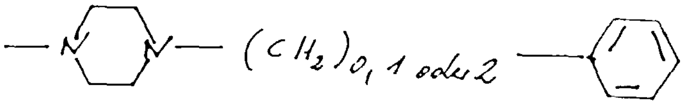

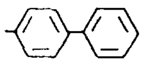

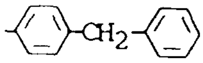

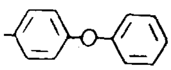

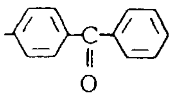

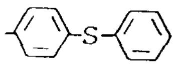

oder

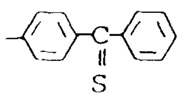

ist,
worin $R^6$ $(C_1\text{-}C_4)$Alkyl, Hydroxy, $-N_3$, Halogen (F, Cl, Br, J), $CF_3$, $NO_2$ oder $(C_1\text{-}C_4)$Alkoxy ist und

v 0, 1, 2 oder 3 ist,

oder deren Salze mit physiologisch verträglichen Säuren oder Komplexbildern.

**4.** Verbindung nach einem der Ansprüche 1 bis 3, worin

$R_4$ eine der folgenden Bedeutungen hat:

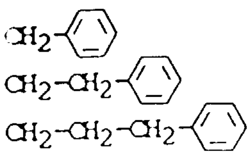

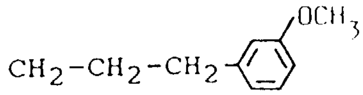

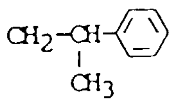

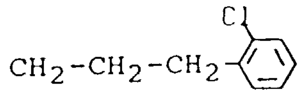

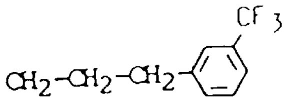

$(CH_2)_4-CH_3$

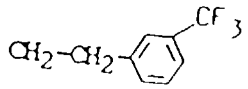

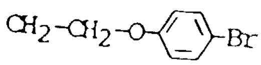

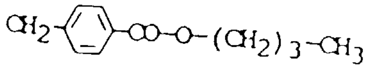

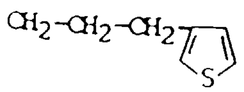

5. Verbindung nach einem der Ansprüche 1 bis 4, worin A 6,7-Dimethoxybenzo ist.

6. Verbindung nach einem der Ansprüche 1 bis 5, worin $R^3$ Phenyl oder Cyclohexyl ist.

7. Verbindung nach einem der Ansprüch 1 bis 6, worin B 0 oder $CH_2$ ist.

8. Verfahren zur Herstellung einer Verbindung nach einem der Ansprüche 1 bis 7, dadurch gekennzeichnet, daß man eine Verbindung der allgemeinen Formel IV

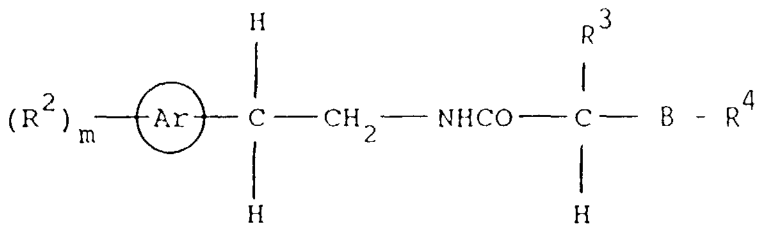

in der $R_2$, $R_3$, $R^{4,}$ m und B, wie in einem der Ansprüche 1 bis 7 definiert sind und Ar Phenyl, Indolyl oder 2- oder 3-Thienyl bedeutet, in Gegenwart eines Kondensationsmittels cyclisiert und gewünschtenfalls in ihr Salz überführt.

9. Pharmazeutische Zubereitung enthaltend eine Verbindung gemäß einem der Ansprüche 1 bis 7.

10. Verwendung einer Verbindung nach einem der Ansprüche 1 bis 7 für die Herstellung einer pharmazeutischen

Zubereitung zur Hirnprotektion, für die Behandlung chronisch inflammatorischer Prozesse, mit antiproliferativer Wirkung oder zur Behandlung der Colitis Ulcerosa oder des Morbus Crohn.

**Claims**

**1.** Compound of general formula I

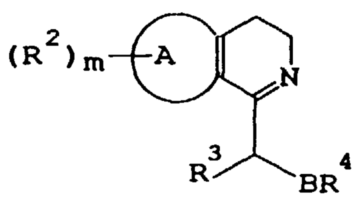

I

wherein

A denotes a benzo, indolo or thieno group; wherein, if A is benzo, m is 2 or 3, and the substituents $R^2$ independently of each other denote hydroxy, $(C_{1-4})$alkoxy, benzyloxy, halogen (F, Cl, Br, I), $(C_{1-4})$alkyl, methanesulphonyloxy or methanesulphonamido, or two adjacent substituents $R^2$ may together represent $-O-CH_2-O-$ or $-O-CH_2-CH_2-O-$; and if A is indolo or thieno, m is zero;

B represents the group -O-, -S- or $-CHR^5-$, wherein $R^5$ is hydrogen, $(C_{1-6})$alkyl, phenyl or benzyl;

$R^3$ denotes 2- or 3-thienyl, $(C_{4-7})$cycloalkyl, $(C_{4-6})$-cycloalkyl$(C_{1-5})$alkyl or

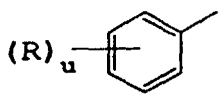

or

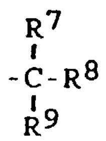

(wherein

R is $(C_{1-4})$alkyl, hydroxy, $-N_3$, halogen (F, Cl, Br, I), $CF_3$ or $(C_{1-4})$alkoxy,

u is 0, 1, 2 or 3, and

$R^7$, $R^8$ and $R^9$ independently of one another may represent methyl, ethyl, propyl, phenyl or benzyl, but not more than two of the substituents can simultaneously represent phenyl or benzyl);

$R^4$ denotes

(a) branched or unbranched $C_{3-6}$-alkenyl which may be substituted by phenyl, or
(b) branched or unbranched $C_{3-6}$-alkynyl which may be substituted by phenyl, or
(c) branched or unbranched $C_{1-13}$-alkyl, wherein the alkyl may be substituted by
hydroxy,
$(C_{1-4})$alkoxy,
di$(C_{1-4})$alkylamino,
furyl,
pyridyl,
pyrrolidinyl, N-methylpyrrolidinyl,
morpholino,
indolyl,
nitrilo,

thienyl,
adamantyl,
cyclohexyl,
phenoxy,
benzyloxy,
naphthyloxy or
phenyl,
(whilst this phenyl or the phenyl contained in the phenoxy group or benzyloxy group may be mono-, di- or trisubstituted by hydroxy, $(C_{1-4})$alkoxy, benzyloxy, halogen (F, Cl, Br, I), $CF_3$, $N_3$, $NO_2$, $(C_{1-4})$alkyl, adamantyl, $-SO_2NH_2$, $NHCOCH_3$, $-NHSO_2CH_3$ or $-C(O)O-R_{14}$,
[wherein $R_{14}$ is $(C_{3-7})$cycloalkyl or branched or unbranched $(C_{1-6})$alkyl, whilst the alkyl may be substituted by phenyl, and this phenyl may be mono- to trisubstituted by halogen (F, Cl, Br, I), $CF_3$, $C_1$- or $C_2$-alkyl, $C_1$- or $C_2$-alkoxy]
or may be substituted by the bridge $-O-CH_2-O-$), or
by 2 unsubstituted phenyl groups;
(d)

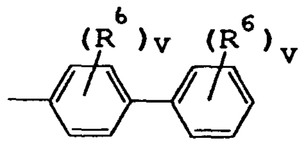

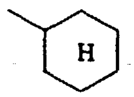

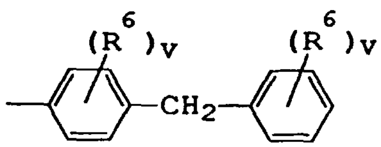

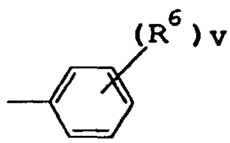

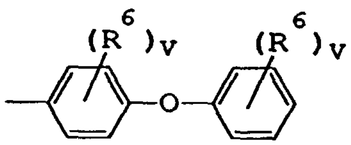

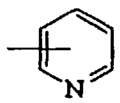

or

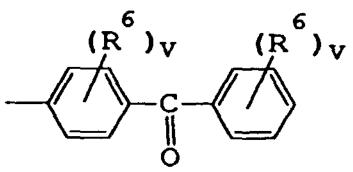

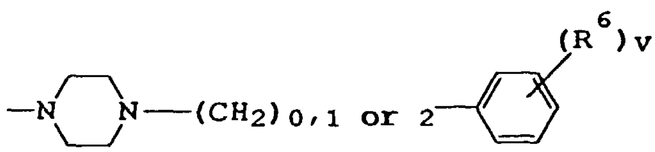

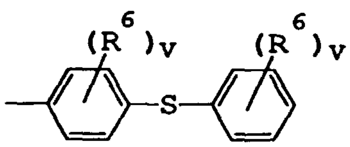

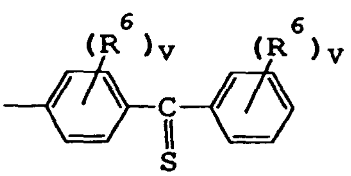

wherein $R^6$ is $(C_{1-4})$alkyl, hydroxy, $-N_3$, halogen (F, Cl, Br, I), $CF_3$, $NO_2$ or $(C_{1-4})$alkoxy and

v is 0, 1, 2 or 3,

or the salts thereof with physiologically acceptable acids or complexing agents.

**2.** Compound according to claim 1, wherein, if A is benzo, m is 2 and the two $R^2$ are in positions 6 and 7.

**3.** Compound according to claim 1 or 2, wherein

A denotes benzo; m is 2, and the substituents $R^2$ independently of one another represent hydroxy, $(C_{1-4})$alkoxy, benzyloxy, halogen (F, Cl, Br, I) or $(C_{1-4})$alkyl, or two adjacent substituents $R^2$ may together represent -O-$CH_2$-O;

B denotes the group -O-, -S- or -$CHR^5$-, wherein $R^5$ is hydrogen, methyl, phenyl or benzyl;

$R^3$ is 2- or 3-thienyl, $(C_{4-7})$cycloalkyl, or

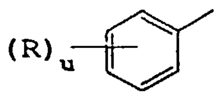 or 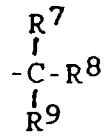

(wherein

R is $(C_{1-4})$alkyl, halogen (F, Cl, Br, I), $CF_3$ or $(C_{1-4})$alkoxy,

u is 0, 1, 2 or 3, and

$R^7$, $R^8$ and $R^9$ independently of one another may represent methyl, ethyl, propyl, phenyl or benzyl, but not more than 2 of the substituents may simultaneously represent phenyl or benzyl);

$R^4$ denotes

(a) branched or unbranched $C_{3-6}$-alkenyl which may be substituted by phenyl or
(b) branched or unbranched $C_{3-6}$-alkynyl which may be substituted by phenyl, or
(c) branched or unbranched $C_{1-13}$-alkyl, wherein the alkyl may be substituted by
thienyl,
adamantyl,
cyclohexyl,
phenoxy,
benzyloxy or
phenyl
(whilst this phenyl or the phenyl contained in the phenoxy group or benzyloxy group may be mono-, di- or trisubstituted by $(C_{1-4})$alkoxy, halogen (F, Cl, Br, I), $CF_3$, $N_3$, $NO_2$, $(C_{1-4})$alkyl, or -C(O)-O-$(CH_2)_{1-3}$-$CH_3$ or by the bridge -O-$CH_2$-O-), or by 2 unsubstituted phenyl groups;
(d)

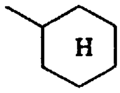

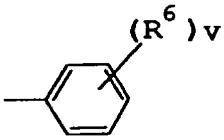

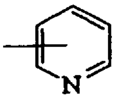

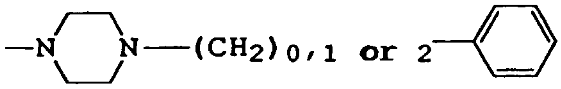

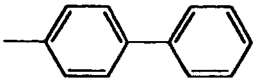

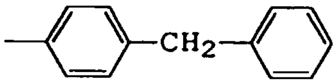

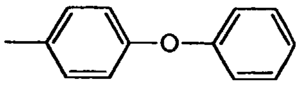

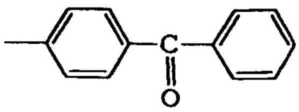

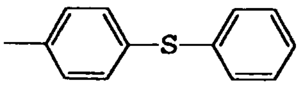

or

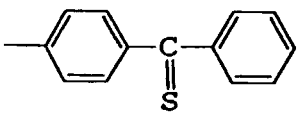

wherein $R^6$ denotes $(C_{1-4})$alkyl, hydroxy, $-N_3$, halogen (F, Cl, Br, I), $CF_3$, $NO_2$ or $(C_{1-4})$alkoxy and

v is 0, 1, 2 or 3,

or the salts thereof with physiologically acceptable acids or complexing agents.

**4.** Compound according to one of claims 1 to 3, wherein $R^4$ has one of the following meanings:

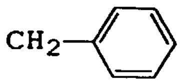

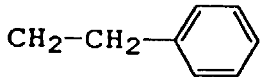

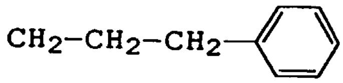

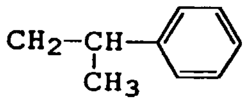

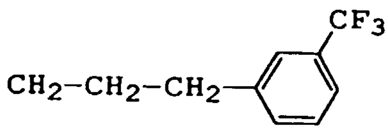

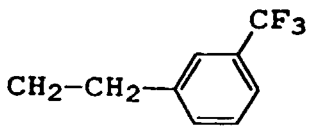

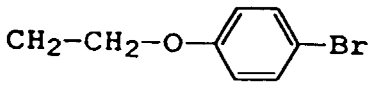

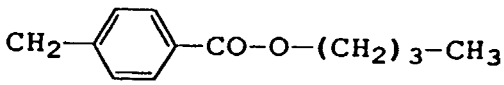

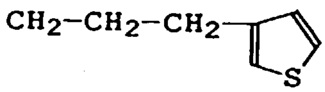

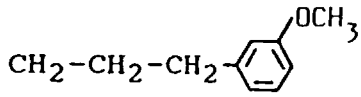

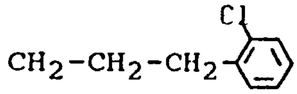

$(CH_2)_4-CH_3$.

5. Compound according to one of claims 1 to 4, wherein A is 6,7-dimethoxybenzo.

6. Compound according to one of claims 1 to 5, wherein $R^3$ is phenyl or cyclohexyl.

7. Compound according to one of claims 1 to 6, wherein B is O or $CH_2$.

8. Process for preparing a compound according to one of claims 1 to 7, characterised in that a compound of general formula IV

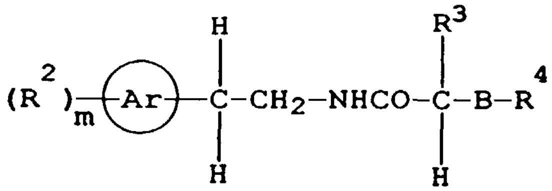

wherein $R^2$, $R^3$, $R^4$, m and B are defined as in one of claims 1 to 7 and Ar denotes phenyl, indolyl or 2- or 3-thienyl, is cyclised in the presence of a condensing agent and, if desired, converted into a salt thereof.

**9.** Pharmaceutical preparation containing a compound according to one of claims 1 to 7.

**10.** Use of a compound according to one of claims 1 to 7 for the production of a pharmaceutical preparation for cerebral protection, for the treatment of chronic inflammatory processes, having an antiproliferative activity or for treating ulcerative colitis or Crohn's disease.

**Revendications**

**1.** Composé de formule générale I

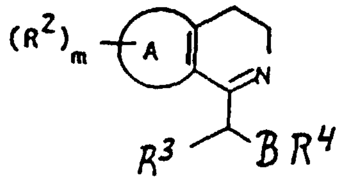

où

A représente un reste benzo, indolo ou thiéno, où, quand A est benzo, m est 2 ou 3 et les substituants $R^2$ peuvent être indépendamment les uns des autres hydroxyle, alcoxy en $C_1$-$C_4$, benzyloxy, halogène (F, Cl, Br, I), alkyle en $C_1$-$C_4$, méthanesulfonyloxy ou méthanesulfonamido, ou deux substituants $R^2$ voisins peuvent être ensemble $-O-CH_2-O-$ ou $-O-CH_2-CH_2-O-$, et quand A est indolo ou thiéno, m est nul;
B représente le groupe -O-, -S- ou $-CHR^5-$ où $R^5$ est l'hydrogène, alkyle en $C_1$-$C_6$, phényle ou benzyle;
$R^3$ représente 2- ou 3-thiényle, cycloalkyle en $C_4$-$C_7$, cycloalkyle en $C_4$-$C_6$-alkyle en $C_1$-$C_5$ ou

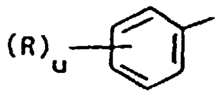

ou

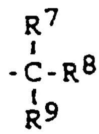

où

R est alkyle en $C_1$-$C_4$, hydroxyle, $-N_3$, halogène (F, Cl, Br, I), $CF_3$ ou alcoxy en $C_1$-$C_4$,
u est 0, 1, 2 ou 3, et
$R^7$, $R^8$ et $R^9$ peuvent être indépendamment les uns des autres méthyle, éthyle, propyle, phényle ou benzyle, pas plus de 2 des substituants pouvant être en même temps phényle ou benzyle;

$R^4$

(a) est alcényle ramifié ou non ramifié de 3 à 6 atomes de carbone, qui peut être substitué par phényle, ou
(b) est alcynyle ramifié ou non ramifié de 3 à 6 atomes de carbone, qui peut être substitué par phényle, ou
(c) est alkyle ramifié ou non ramifié de 1-13 atomes de carbone, où l'alkyle peut être substitué par
hydroxyle,
alcoxy en $C_1$-$C_4$,
dialkyle en $C_1$-$C_4$-amino,
furyle,
pyridyle,
pyrrolidinyle, N-méthylpyrrolidinyle,
morpholino,
indolyle,
nitrilo,
thiényle,
adamantyle,

cyclohexyle,
phénoxy,
benzyloxy,
naphtyloxy ou
phényle,
(où ce phényle ou le phényle contenu dans le groupe phénoxy ou le groupe benzyloxy peut être substitué une, deux ou trois fois par hydroxyle, alcoxy en $C_1$-$C_4$, benzyloxy, halogène (F, Cl, Br, I), $CF_3$, $N_3$, $NO_2$, alkyle en $C_1$-$C_4$, adamantyle, $-SO_2NH_2$, $NHCOCH_3$, $-NHSO_2CH_3$ ou -C(O)O-$R_{14}$ [où $R_{14}$ est cycloalkyle en $C_3$-$C_7$ ou alkyle ramifié ou non ramifié en $C_1$-$C_6$, où l'alkyle peut être substitué par phényle, ce phényle pouvant être substitué une à trois fois par halogène (F, Cl, Br, I), $CF_3$, alkyle en $C_1$ ou $C_2$ ou alcoxy en $C_1$ ou $C_2$] ou par le pont $-O-CH_2-O-$),
ou
par deux groupes phényle non substitués,

(d) est

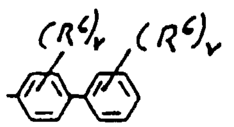

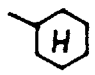

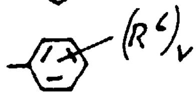

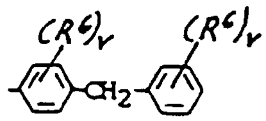

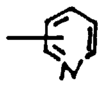

ou

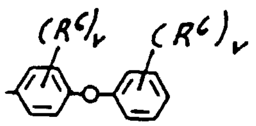

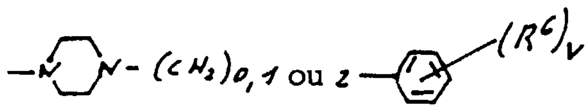

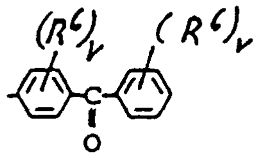

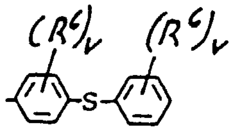

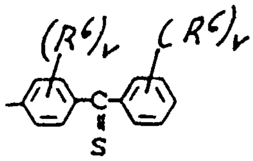

où $R^6$ est alkyle en $C_1$-$C_4$, hydroxyle, $-N_3$, halogène (F, Cl, Br, I), $CF_3$, $NO_2$ ou alcoxy en $C_1$-$C_4$ et

v est 0, 1, 2 ou 3,
ou ses sels avec des acides ou des complexants physiologiquement acceptables.

**2.** Composé selon la revendication 1 où, quand A est benzo, m est 2 et les deux $R^2$ sont dans les positions 6 et 7.

**3.** Composé selon la revendication 1 ou 2 où

A représente benzo, m est 2, et les substituants $R^2$ peuvent représenter indépendamment l'un de l'autre hydroxyle, alcoxy en $C_1$-$C_4$, benzyloxy, halogène (F, Cl, Br, I) ou alkyle en $C_1$-$C_4$, ou deux substituants $R^2$ voisins peuvent être ensemble -O-$CH_2$-O-;

B représente le groupe -O-, -S- ou -$CHR^5$-, où $R^5$ est l'hydrogène, méthyle, phényle ou benzyle;

$R^3$ représente 2- ou 3-thiényle, cycloalkyle en $C_4$-$C_7$ ou

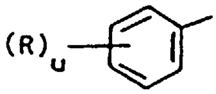

ou

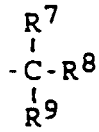

où

R est alkyle en $C_1$-$C_4$, halogène (F, Cl, Br, I), $CF_3$ ou alcoxy en $C_1$-$C_4$,

u est 0, 1, 2 ou 3, et

$R^7$, $R^8$ et $R^9$ peuvent être indépendamment les uns des autres méthyle, éthyle, propyle, phényle ou benzyle, pas plus de deux des substituants pouvant être en même temps phényle ou benzyle;

$R^4$

(a) est alcényle ramifié ou non ramifié de 3 à 6 atomes de carbone, qui peut être substitué par phényle, ou

(b) est alcynyle ramifié ou non ramifié de 3 à 6 atomes de carbone, qui peut être substitué par phényle, ou

(c) est alkyle ramifié ou non ramifié de 1-13 atomes de carbone, où l'alkyle peut être substitué par

thiényle,

adamantyle,

cyclohexyle,

phénoxy,

benzyloxy

ou

phényle,

(où ce phényle ou le phényle contenu dans le groupe phénoxy ou le groupe benzyloxy peut être substitué une, deux ou trois fois par alcoxy en $C_1$-$C_4$, halogène (F, Cl, Br, I), $CF_3$, $N_3$, $NO_2$, alkyle en $C_1$-$C_4$ ou -C(O)-O-$(CH_2)_{1\text{-}3}$-$CH_3$ ou par le pont -O-$CH_2$-O-),

ou

par deux groupes phényle non substitués,

(d) est

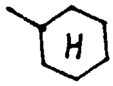

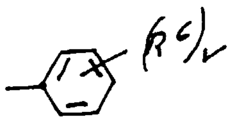

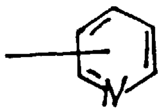

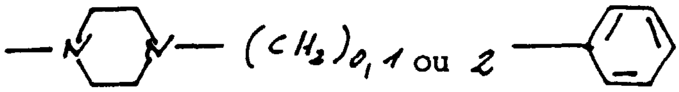

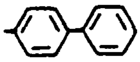

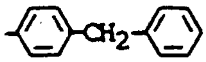

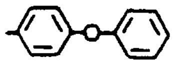

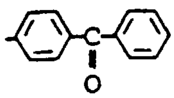

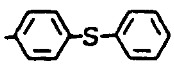

ou

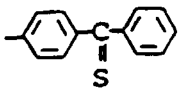

où $R^6$ est alkyle en $C_1$-$C_4$, hydroxyle, $-N_3$, halogène (F, Cl, Br, I), $CF_3$, $NO_2$ ou alcoxy en $C_1$-$C_4$ et

v est 0, 1, 2 ou 3,
ou ses sels avec des acides ou des complexants physiologiquement acceptables.

**4.** Composé selon l'une des revendications 1 à 3 où

$R^4$ a l'une des significations suivantes:

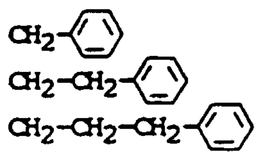

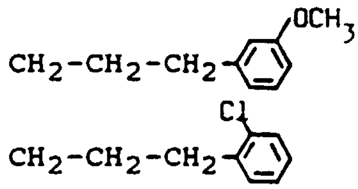

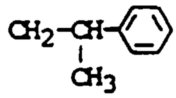

$(CH_2)_4-CH_3$

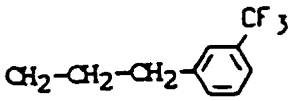

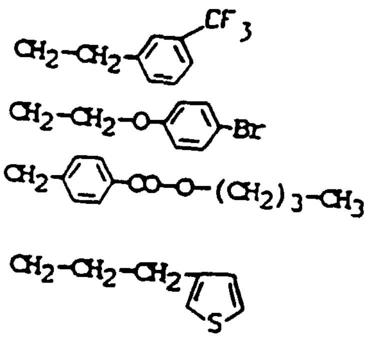

**5.** Composé selon l'une des revendications 1 à 4 où A est 6,7-diméthoxybenzo.

**6.** Composé selon l'une des revendications 1 à 5 où $R^3$ est phényle ou cyclohexyle.

**7.** Composé selon l'une des revendications 1 à 6 où B est O ou $CH_2$.

**8.** Procédé de préparation d'un composé selon l'une des revendications 1 à 7 caractérisé en ce que l'on cyclise un composé de formule générale IV

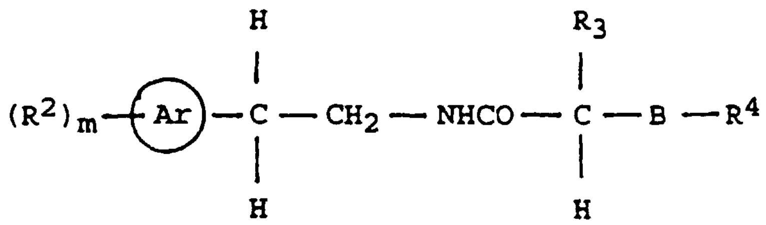

dans laquelle $R^2$, $R^3$, $R^4$, m et B sont définis comme dans l'une des revendications 1 à 7 et Ar représente phényle, indolyle ou 2- ou 3-thiényle, en présence d'un agent de condensation et on le convertit si on le souhaite en son sel.

**9.** Préparation pharmaceutique contenant un composé selon l'une des revendications 1 à 7.

**10.** Utilisation d'un composé selon l'une des revendications 1 à 7 pour la production d'une préparation pharmaceutique pour la protection cérébrale, pour le traitement des processus inflammatoires chroniques, à effet antiprolifératif ou pour le traitement de la colite ulcéreuse ou de la maladie de Crohn.